(19) Europäisches Patentamt
European Patent Office
Office européen des brevets

(11) EP 2 729 151 B1

(12) **EUROPEAN PATENT SPECIFICATION**

(45) Date of publication and mention of the grant of the patent:
**31.03.2021 Bulletin 2021/13**

(21) Application number: **12730993.8**

(22) Date of filing: **03.07.2012**

(51) Int Cl.:
***A61K 31/7004*** *(2006.01)* ***A61P 13/00*** *(2006.01)*

(86) International application number:
**PCT/EP2012/062922**

(87) International publication number:
**WO 2013/007557 (17.01.2013 Gazette 2013/03)**

(54) **PHARMACEUTICAL COMPOSITION, METHODS FOR TREATING AND USES THEREOF**

PHARMAZEUTISCHE ZUSAMMENSETZUNG, VERFAHREN ZUR BEHANDLUNG UND VERWENDUNG

COMPOSITION PHARMACEUTIQUE, PROCÉDÉS DESTINÉS À UN TRAITEMENT ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2011 US 201161505598 P**

(43) Date of publication of application:
**14.05.2014 Bulletin 2014/20**

(60) Divisional application:
**21157166.6**

(73) Proprietor: **Boehringer Ingelheim International GmbH**
**55216 Ingelheim am Rhein (DE)**

(72) Inventors:
- **BROEDL, Uli**
  **55216 Ingelheim Am Rhein (DE)**
- **VON EYNATTEN, Maximilian**
  **55216 Ingelheim Am Rhein (DE)**
- **JOHANSEN, Odd-Erik**
  **55216 Ingelheim Am Rhein (DE)**
- **KLEIN, Thomas**
  **55216 Ingelheim Am Rhein (DE)**
- **LUIPPOLD, Gerd**
  **55216 Ingelheim Am Rhein (DE)**

(74) Representative: **Simon, Elke Anna Maria et al**
**Boehringer Ingelheim GmbH**
**Binger Strasse 173**
**55216 Ingelheim am Rhein (DE)**

(56) References cited:
**WO-A2-2008/055940**

- **VALLON VOLKER ET AL: "Glomerular hyperfiltration in experimental diabetes mellitus: Potential role of tubular reabsorption", JOURNAL OF THE AMERICAN SOCIETY OF NEPHROLOGY, vol. 10, no. 12, December 1999 (1999-12), pages 2569-2576, XP009161748, ISSN: 1046-6673**
- **YAO CHUN-HSU ET AL: "Discovery of Novel N-beta-D-Xylosylindole Derivatives as Sodium-Dependent Glucose Cotransporter 2 (SGLT2) Inhibitors for the Management of Hyperglycemia in Diabetes", JOURNAL OF MEDICINAL CHEMISTRY, vol. 54, no. 1, January 2011 (2011-01), pages 166-178, XP009161759, ISSN: 0022-2623**
- **CHERNEY DAVID Z I ET AL: "Renal Hemodynamic Effect of Sodium-Glucose Cotransporter 2 Inhibition in Patients With Type 1 Diabetes Mellitus", CIRCULATION, vol. 129, no. 5, February 2014 (2014-02), pages 587-597, ISSN: 0009-7322(print)**
- **R Grempler ET AL: "Empagliflozin, a novel selective sodium glucose cotransporter-2 (SGLT-2) inhibitor: characterisation and comparison with other SGLT-2 inhibitors Introduction", Diabetes Obesity and Metabolism June, vol. 14, 1 January 2012 (2012-01-01), pages 83-90, XP055228524, DOI: 10.1111/j.1463-1326.2011.01517.x**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field of the Invention

The invention relates to a pharmaceutical composition comprising an SGLT2 inhibitor for use in a method for preventing, slowing the progression of, delaying or treating renal hyperfiltrative injury in a patient diagnosed with type 1 diabetes mellitus. Furthermore the invention relates to a pharmaceutical composition comprising the SGLT2 inhibitor 1-chloro-4-(β-D-glucopyranos-1-yl)-2-[4-((*S*)-tetrahydrofuran-3-yloxy)-benzyl]-benzene for use in a method for preventing, slowing the progression of, delaying or treating renal hyperfiltration in a patient diagnosed with type 1 diabetes mellitus.

### Background of the Invention

Kidneys are bean-shaped organs, located near the middle of the back. Inside each kidney about a million tiny structures called nephrons filter blood. They remove waste products and extra water, which become urine. Damage to the nephrons represents one form of kidney disease. This damage may leave kidneys unable to remove wastes. Some damage, e.g. damage related to hyperfiltration can occur slowly over years, initially often without obvious symptoms.

The 'hyperfiltrative hypothesis' implies that the excess demand on a limited renal reserve produces adaptive and ultimately pathologic changes in the kidney which finally lead to 'nephron exhaustion'. At the single-nephron level, hyperfiltration is hypothesized to be an early link in the chain of events that lead from intraglomerular hypertension to albuminuria and, subsequently, to reduced Glomerular Filtration Rate (GFR). Based on this hypothesis such a finding therefore represents a risk for subsequent renal injury and could be classified as an early manifestation of renal damage often referred to as the hyperfiltrative stage. Such renal hyperfiltration can lead to early glomerular lesions and to microalbuminuria, which itself can lead to macroalbuminuria and to end-stage renal disease.

The influence of hyperfiltration on renal function decline has been most thoroughly evaluated in kidney transplant recipients and donors, and in patients uninephrectomized for acquired renal disease, but also in patients with diabetes mellitus (Magee et al. Diabetologia 2009; 52: 691-697). In theory, any reduction in functional nephron number will lead to adaptive glomerular hypertension and hyperfiltration whether induced genetically, surgically, or by acquired renal disease. Moreover, hyperfiltration has been shown to occur in certain pathophysiologic conditions even when renal mass is intact, e.g. in diabetes.

Therefore, there is a medical need for methods, medicaments and pharmaceutical compositions with a good efficacy with regard to renal hyperfiltrative injury.

Vallon et al., Journal of the American Society of Nephrology, vol. 10, no. 12, 1999, pages 2569-2576, describe glomerular hyperfiltration in experimental diabetes mellitus and the role of tubular reabsorption. To test for a role of the Na+/glucose cotransporter in the increased tubular reabsorption, the reduced electrolyte concentration in early distal tubular fluid, and glomerular hyperfiltration in rats, phlorizin, an inhibitor of Na+/glucose cotransport, was applied into the early proximal tubule of control rats and rats with streptozotocin-induced experimental diabetes.

WO 2008/055940 A2 relates to pharmaceutical compositions comprising a SGLT2 inhibitor and an antidiabetic agent selected from a group as specified.

Yao et al., J. Med. Chem. 2011, 54, 166-178 describe the discovery of novel N-beta-D-xylosylindole derivatives as SGLT2 inhibitors for the management of hyperglycemia in diabetes.

### Summary of the Invention

The present invention provides a pharmaceutical composition comprising the SGLT2 inhibitor 1-chloro-4-(β-D-glucopyranos-1-yl)-2-[4-((*S*)-tetrahydrofuran-3-yloxy)-benzyl]-benzene for use in a method for preventing, slowing the progression of, delaying or treating renal hyperfiltrative injury in a patient diagnosed with type 1 diabetes mellitus in need thereof.

In one aspect, the patient is an individual diagnosed or showing one or more of the following conditions:

(a) diabetes mellitus;
(b)congenital or acquired obstructive uro/nephropathy;
(c) progressive chronic kidney disease (CKD);
(d) acute renal failure (ARF);
(e) renal transplant recipients;
(f) renal transplant donors; or
(g) unilateral total or partial nephrectomized patients.

In one aspect, the patient has an GFR equal to or greater than 125 mL/min/1.73 $m^2$. In a further aspect, the

patient has an GFR equal to or greater than 140 mL/min/1.73 $m^2$.

In one aspect, the patient:

(1) is an individual diagnosed of one or more of the conditions selected from the group consisting of overweight, obesity, visceral obesity and abdominal obesity; or

(2) is an individual who shows one, two or more of the following conditions:

(a) a fasting blood glucose or serum glucose concentration greater than 100 mg/dL, in particular greater than 125 mg/dL;

(b) a postprandial plasma glucose equal to or greater than 140 mg/dL;

(c) an HbA1c value equal to or greater than 6.0 %, in particular equal to or greater than 6.5%, in particular equal to or greater than 8.0 %; or

(3) is an individual wherein one, two, three or more of the following conditions are present:

(a) obesity, visceral obesity and/or abdominal obesity,

(b) triglyceride blood level ≥ 150 mg/dL,

(c) HDL-cholesterol blood level < 40 mg/dL in female patients and < 50 mg/dL in male patients,

(d) a systolic blood pressure ≥ 130 mm Hg and a diastolic blood pressure ≥ 85 mm Hg,

(e) a fasting blood glucose level ≥ 100 mg/dL; or

(4) is an individual with morbid obesity.

In one aspect, the pharmaceutical composition additionally comprises one or more pharmaceutically acceptable carriers. In one aspect, the pharmaceutical composition comprises the SGLT-2 inhibitor in a range from about 10 mg to 25 mg. In one aspect, the pharmaceutical composition comprises 10 mg or 25 mg of the SGLT-2 inhibitor.

In a further embodiment, the present invention provides a pharmaceutical composition comprising the SGLT2 inhibitor 1-chloro-4-(β-D-glucopyranos-1-yl)-2-[4-((*S*)-tetrahydrofuran-3-yloxy)-benzyl]-benzene for use in a method for preventing, slowing the progression of, delaying or treating renal hyperfiltration in a patient diagnosed with type 1 diabetes mellitus.

In one aspect, the patient is an individual diagnosed or showing one or more of the following conditions:

(a) diabetes mellitus;

(b)congenital or acquired obstructive uro/nephropathy;

(c) progressive chronic kidney disease (CKD);

(d) acute renal failure (ARF);

(e) renal transplant recipients;

(f) renal transplant donors; or

(g) unilateral total or partial nephrectomized patients.

In one aspect, the patient has an GFR equal to or greater than 125 mL/min/1.73 $m^2$. In a further aspect, the patient has an GFR equal to or greater than 140 mL/min/1.73 $m^2$.

In one aspect, the patient:

(1) is an individual diagnosed of one or more of the conditions selected from the group consisting of overweight, obesity, visceral obesity and abdominal obesity; or

(2) is an individual who shows one, two or more of the following conditions:

(a) a fasting blood glucose or serum glucose concentration greater than 100 mg/dL, in particular greater than 125 mg/dL;

(b) a postprandial plasma glucose equal to or greater than 140 mg/dL;

(c) an HbA1c value equal to or greater than 6.0 %, in particular equal to or greater than 6.5%, in particular equal to or greater than 8.0 %;

(3) is an individual wherein one, two, three or more of the following conditions are present:

(a) obesity, visceral obesity and/or abdominal obesity,

(b) triglyceride blood level ≥ 150 mg/dL,

(c) HDL-cholesterol blood level < 40 mg/dL in female patients and < 50 mg/dL in male patients,
(d) a systolic blood pressure ≥ 130 mm Hg and a diastolic blood pressure ≥ 85 mm Hg,
(e) a fasting blood glucose level ≥ 100 mg/dL; or

(4) is an individual with morbid obesity.

In one aspect, the pharmaceutical composition additionally comprises one or more pharmaceutically acceptable carriers. In one aspect, the pharmaceutical composition comprises the SGLT-2 inhibitor in a range from about 10 mg to 25 mg. In one aspect, the pharmaceutical composition comprises 10 mg or 25 mg of the SGLT-2 inhibitor.

**Definitions**

The term **"active ingredient"** of a pharmaceutical composition according to the present invention means the SGLT2 inhibitor according to the present invention. An "active ingredient is also sometimes referred to herein as an "active substance".

The term **"hyperfiltration"** is defined as an elevation in the filtration rate of the renal glomeruli. In one aspect, hyperfiltration is defined as a whole kidney filtration rate equal to or greater than 125 mL/min/1.73 $m^2$, especially equal to or greater than 140 mL/min/1.73 $m^2$, as measured using a method described hereinbelow. Hyperfiltration may also be defined as related to an absolute GFR greater to the 90$^{th}$, or the 95$^{th}$, percentile in the studied population after adjusting for sex, age, weight, height, and the use of ACE inhibitors or ARB (see Melsom et al. Diabetes Care 2011; DOI: 10.2337/dc11-0235).

The term **"glomerular filtration rate (GFR)"** is defined as the volume of fluid filtered from the renal (kidney) glomerular capillaries into the Bowman's capsule per unit time. It is indicative of overall kidney function. The glomerular filtration rate (GFR) can be calculated by measuring any chemical that has a steady level in the blood, and is freely filtered but neither reabsorbed nor secreted by the kidneys. The rate therefore measured is the quantity of the substance in the urine that originated from a calculable volume of blood. The GFR is typically recorded in units of *volume per time,* e.g., milliliters per minute and the formula below can be used:

$$\textbf{GFR} = (\textbf{Urine Concentration} \times \textbf{Urine Volume}) / \textbf{Plasma Concentration}$$

The GFR can be determined by injecting inulin into the plasma. Since inulin is neither reabsorbed nor secreted by the kidney after glomerular filtration, its rate of excretion is directly proportional to the rate of filtration of water and solutes across the glomerular filter. A normal value is: GFR = 90-125 mL/min/1.73 $m^2$, in particular GFR = 100-125 mL/min/1.73 $m^2$.

Other principles to determine GFR involve measuring 51Cr-EDTA, [125I]iothalamate or iohexol.

The **"estimated glomerular filtration rate (eGFR)"** is defined as derived at screening from serum creatinine values based on e.g., the Chronic Kidney Disease Epidemiology Collaboration (CKD-EPI) equation, the Cockcroft-Gault formula or the Modification of Diet in Renal Disease (MDRD) formula, which are all known in the art.

Subjects with normal renal function are defined as eGFR ≥90 ml/min. Subjects with mild impairment of renal function as defined eGFR ≥60 and <90 ml/min). Subjects with moderate impairment as defined as eGFR ≥30 and <60 ml/min). Subjects with severe impairment as defined as eGFR ≥15 and <30 ml/min.

The term **"renal hyperfiltrative injury"** is defined as a manifestation of renal damage caused predominantly by renal hyperfiltration, which often is an early link in the chain of events to further renal injury, acknowledging that hyperfiltration often works in concert with other chronic kidney disease risk factors in the pathogenesis of renal injury.

The term **"body mass index"** or **"BMI"** of a human patient is defined as the weight in kilograms divided by the square of the height in meters, such that BMI has units of kg/$m^2$.

The term **"overweight"** is defined as the condition wherein the adult individual of Europide origin has a BMI greater than or 25 kg/$m^2$ and less than 30 kg/$m^2$. In subjects of Asian origin the term "overweight" is defined as the condition wherein the adult individual has a BMI greater than or 23 kg/$m^2$ and less than 25 kg/$m^2$. The terms "overweight" and "pre-obese" are used interchangeably.

The term **"obesity"** is defined as the condition wherein the adult individual of Europid origin has a BMI equal to or greater than 30 kg/$m^2$. According to a WHO definition the term obesity may be categorized as follows: the term "class I obesity" is the condition wherein the BMI is equal to or greater than 30 kg/$m^2$ but lower than 35 kg/$m^2$; the term "class II obesity" is the condition wherein the BMI is equal to or greater than 35 kg/$m^2$ but lower than 40 kg/$m^2$; the term "class III obesity" is the condition wherein the BMI is equal to or greater than 40 kg/$m^2$. In subjects of Asian origin the term "obesity" is defined as the condition wherein the adult individual has a BMI equal or greater than 25 kg/$m^2$. Obesity in Asians may be categorized further as follows: the term "class I obesity" is the condition wherein the BMI is equal to

or greater than 25 $kg/m^2$ but lower than 30 $kg/m^2$; the term "class II obesity" is the condition wherein the BMI is equal to or greater than 30 $kg/m^2$.

The term **"visceral obesity"** is defined as the condition wherein a waist-to-hip ratio of greater than or equal to 1.0 in men and 0.8 in women is measured. It defines the risk for insulin resistance and the development of pre-diabetes.

The term **"abdominal obesity"** is usually defined as the condition wherein the waist circumference is > 40 inches or 102 cm in men, and is > 35 inches or 94 cm in women (for normal ranges of popupations, see for example "Joint scientific statement (IDF, NHLBI, AHA, WHO, IAS, IASO). Circulation 2009; 120:1640-1645"). With regard to a Japanese ethnicity or Japanese patients abdominal obesity may be defined as waist circumference ≥ 85 cm in men and ≥ 90 cm in women (see e.g. investigating committee for the diagnosis of metabolic syndrome in Japan).

The term **"morbid obesity"** is defined herein as a condition in which the individual of Europid origin has a BMI > 40 or has a BMI > 35 and a comorbidity such as diabetes melitus or hypertension (see World Health Organization. Obesity: Preventing and Managing the Global Epidemic: Report on a WHO Consultation. World Health Organ Tech Rep Ser. 2000; 894: i-xii, 1-253).

The term **"euglycemia"** is defined as the condition in which a subject has a fasting blood glucose concentration within the normal range, greater than 70 mg/dL (3.89 mmol/L) and less than 100 mg/dL (5.6 mmol/L), and a 2h postprandial glucose concentration less than 140 mg/dl. The word "fasting" has the usual meaning as a medical term.

The term **"hyperglycemia"** is defined as the condition in which a subject has a fasting blood glucose concentration above the normal range, greater than 100 mg/dL (5.6 mmol/L). The word "fasting" has the usual meaning as a medical term.

The term **"hypoglycemia"** is defined as the condition in which a subject has a blood glucose concentration below the normal range, in particular below 70 mg/dL (3.89 mmol/L).

The term **"postprandial hyperglycemia"** is defined as the condition in which a subject has a 2 hour postprandial blood glucose or serum glucose concentration greater than 200 mg/dL (11.11 mmol/L).

The term **"impaired fasting blood glucose"** or **"IFG"** is defined as the condition in which a subject has a fasting blood glucose concentration or fasting serum glucose concentration in a range from 100 to 125 mg/dl (i.e. from 5.6 to 6.9 mmol/l. A subject with "normal fasting glucose" has a fasting glucose concentration smaller than 100 mg/dl, i.e. smaller than 5.6 mmol/l.

The term **"impaired glucose tolerance"** or **"IGT"** is defined as the condition in which a subject has a 2 hour postprandial blood glucose or serum glucose concentration greater than 140 mg/dl (7.78 mmol/L) and less than 200 mg/dL (11.11 mmol/L). The abnormal glucose tolerance, i.e. the 2 hour postprandial blood glucose or serum glucose concentration can be measured as the blood sugar level in mg of glucose per dL of plasma 2 hours after taking 75 g of glucose after a fast. A subject with "normal glucose tolerance" has a 2 hour postprandial blood glucose or serum glucose concentration smaller than 140 mg/dl (7.78 mmol/L).

The term **"hyperinsulinemia"** is defined as the condition in which a subject with insulin resistance, with or without euglycemia, has fasting or postprandial serum or plasma insulin concentration elevated above that of normal, lean individuals without insulin resistance, having a waist-to-hip ratio < 1.0 (for men) or < 0.8 (for women).

The terms "insulin-sensitizing", "insulin resistance-improving" or "insulin resistance-lowering" are synonymous and used interchangeably.

The term **"insulin resistance"** is defined as a state in which circulating insulin levels in excess of the normal response to a glucose load are required to maintain the euglycemic state (Ford ES, et al. JAMA. (2002) 287:356-9). A method of determining insulin resistance is the euglycaemic-hyperinsulinaemic clamp test. The ratio of insulin to glucose is determined within the scope of a combined insulin-glucose infusion technique. There is found to be insulin resistance if the glucose absorption is below the 25th percentile of the background population investigated (WHO definition). Rather less laborious than the clamp test are so called minimal models in which, during an intravenous glucose tolerance test, the insulin and glucose concentrations in the blood are measured at fixed time intervals and from these the insulin resistance is calculated. With this method, it is not possible to distinguish between hepatic and peripheral insulin resistance.

Furthermore, insulin resistance, the response of a patient with insulin resistance to therapy, insulin sensitivity and hyperinsulinemia may be quantified by assessing the "homeostasis model assessment to insulin resistance (HOMA-IR)" score, a reliable indicator of insulin resistance (Katsuki A, et al. Diabetes Care 2001; 24: 362-5). Further reference is made to methods for the determination of the HOMA-index for insulin sensitivity (Matthews et al., Diabetologia 1985, 28: 412-19), of the ratio of intact proinsulin to insulin (Forst et al., Diabetes 2003, 52(Suppl.1): A459) and to an euglycemic clamp study. In addition, plasma adiponectin levels can be monitored as a potential surrogate of insulin sensitivity. The estimate of insulin resistance by the homeostasis assessment model (HOMA)-IR score is calculated with the formula (Galvin P, et al. Diabet Med 1992;9:921-8):

$$\text{HOMA-IR} = [\text{fasting serum insulin } (\mu U/mL)] \times [\text{fasting plasma glucose}(mmol/L)/22.5]$$

As a rule, other parameters are used in everyday clinical practice to assess insulin resistance. Preferably, the patient's triglyceride concentration is used, for example, as increased triglyceride levels correlate significantly with the presence of insulin resistance.

Patients with a predisposition for the development of IGT or IFG or type 2 diabetes are those having euglycemia with hyperinsulinemia and are by definition, insulin resistant. A typical patient with insulin resistance is usually overweight or obese. If insulin resistance can be detected, this is a particularly strong indication of the presence of pre-diabetes. Thus, it may be that in order to maintain glucose homoeostasis a person needs 2-3 times as much insulin as a healthy person, without this resulting in any clinical symptoms.

The methods to investigate the **function of pancreatic beta-cells** are similar to the above methods with regard to insulin sensitivity, hyperinsulinemia or insulin resistance: An improvement of beta-cell function can be measured for example by determining a HOMA-index for beta-cell function *(*Matthews et al., Diabetologia 1985, 28: 412-19)*,* the ratio of intact proinsulin to insulin *(*Forst et al., Diabetes 2003, 52(Suppl.1): A459)*,* the insulin/C-peptide secretion after an oral glucose tolerance test or a meal tolerance test, or by employing a hyperglycemic clamp study and/or minimal modeling after a frequently sampled intravenous glucose tolerance test (Stumvoll et al., Eur J Clin Invest 2001, 31: 380-81*).*

The term **"pre-diabetes"** is the condition wherein an individual is pre-disposed to the development of type 2 diabetes. Pre-diabetes extends the definition of impaired glucose tolerance to include individuals with a fasting blood glucose within the high normal range ≥ 100 mg/dL (J. B. Meigs, et al. Diabetes 2003; 52:1475-1484) and fasting hyperinsulinemia (elevated plasma insulin concentration). The scientific and medical basis for identifying pre-diabetes as a serious health threat is laid out in a Position Statement entitled "The Prevention or Delay of Type 2 Diabetes" issued jointly by the American Diabetes Association and the National Institute of Diabetes and Digestive and Kidney Diseases (Diabetes Care 2002; 25:742-749).

Individuals likely to have insulin resistance are those who have two or more of the following attributes: 1) overweight or obese, 2) high blood pressure, 3) hyperlipidemia, 4) one or more $1^{st}$ degree relative with a diagnosis of IGT or IFG or type 2 diabetes. Insulin resistance can be confirmed in these individuals by calculating the HOMA-IR score. For the purpose of this invention, insulin resistance is defined as the clinical condition in which an individual has a HOMA-IR score > 4.0 or a HOMA-IR score above the upper limit of normal as defined for the laboratory performing the glucose and insulin assays, or values above the $75^{th}$ percentile of the reference population.

The term **"type 2 diabetes"** is defined as the condition in which a subject has a fasting (i.e., no caloric intake for 8 hours) blood glucose or serum glucose concentration greater than 125 mg/dL (6.94 mmol/L), when measured at minimum two independent occasions. The measurement of blood glucose values is a standard procedure in routine medical analysis. Type 2 diabetes is also defined as the condition in which a subject has HbA1c equal to, or greater than 6.5%, a two hour plasma glucose equal to, or greater than 200 g/dL (11.1 mmol/L) during an oral glucose tolerance test (OGTT) or a random glucose concentration equal to, or greater than 200 mg/dL (11.1 mmol/L) in conjunction with classic symptoms of hyperglycaemia or hyperglycaemic crisis. In the absence of unequicoval hyperglycaemia, as with most diagnostic tests, a test result diagnostic of diabetes should be repeated to rule out laboratory error. The assessment of HbA1c should be performed using a method certified by the National Glycohemoglobin Standardization Program (NGSP) and standardized or traceable to the Diabetes Control and Complications Trial (DCCT) reference assay. If a OGTT is carried out, the blood sugar level of a diabetic will be in excess of 200 mg of glucose per dL (11.1 mmol/l) of plasma 2 hours after 75 g of glucose have been taken on an empty stomach. In a glucose tolerance test 75 g of glucose are administered orally to the patient being tested after aminimun of 8 hours, typicaly after 10-12 hours, of fasting and the blood sugar level is recorded immediately before taking the glucose and 1 and 2 hours after taking it. In a healthy subject, the blood sugar level before taking the glucose will be between 60 and 110 mg per dL of plasma, less than 200 mg per dL 1 hour after taking the glucose and less than 140 mg per dL after 2 hours. If after 2 hours the value is between 140 and 200 mg, this is regarded as abnormal glucose tolerance.

The term **"late stage type 2 diabetes mellitus"** includes patients with a long-standing duration of diabetes, secondary drug failure, indication for insulin therapy and potentially progression to micro- and macrovascular complications e.g. diabetic nephropathy, or coronary heart disease (CHD).

The term **"type 1 diabetes"** is defined as the condition in which a subject has, in the presence of autoimmunity towards the pancreatic beta-cell (i.e. detection of circulating islet cell autoantibodies ["type 1A diabetes mellitus"], i.e., at least one of: GAD65 [glutamic acid decarboxylase-65], ICA [islet-cell cytoplasm], IA-2 [intracytoplasmatic domain of the tyrosine phosphatase-like protein IA-2], ZnT8 [zinc-transporter-8] or anti-insulin; or other signs of automimmunity without the presence of typical circulating autoantibodies [type 1B diabetes], i.e. as detected through pancreatic biopsy or imaging), a fasting (i.e., no caloric intake for 8 hours) blood glucose or serum glucose concentration greater than 125 mg/dL (6.94 mmol/L). Type 1 diabetes is also defined as the condition in which a subject has, in the presence of autoimmunity towards the pancreatic beta-cell, HbA1c equal to, or greater than 6.5%, a two hour plasma glucose equal

to, or greater than 200 g/dL (11.1 mmol/L) during an oral glucose tolerance test (OGTT) or a random glucose equal to, or greater than 200 mg/dL (11.1 mmol/L) in conjunction with classic symptoms of hyperglycaemia or hyperglycaemic crisis. In the absence of uneqicoval hyperglycaemia, as with most diagnostic tests, a test result diagnostic of diabetes should be repeated to rule out laboratory error. The measurement of blood glucose values is a standard procedure in routine medical analysis.The assessment of HbA1c should be performed using a method certified by the National Glycohemoglobin Standardization Program (NGSP) and standardized or traceable to the Diabetes Control and Complications Trial (DCCT) reference assay. If a OGTT is carried out, the blood sugar level of a diabetic will be in excess of 200 mg of glucose per dL (11.1 mmol/l) of plasma 2 hours after 75 g of glucose have been taken on an empty stomach, in the presence of autoimmunity towards the pancreatic beta cell. In a glucose tolerance test 75 g of glucose are administered orally to the patient being tested after a minimum of 8 hours, typically, 10-12 hours, of fasting and the blood sugar level is recorded immediately before taking the glucose and 1 and 2 hours after taking it. Typically a genetic predisposition is present (e.g. HLA, *INS* VNTR and *PTPN22),* but this is not always the case.

The term **"MODY"** ("maturity onset diabetes of the youth") describes a monogenic form for diabetes that, according to gene affects, is split into MODY variants, e.g., MODY 1,2.3.4 etc.

The term **"LADA"** ("latent autoimmune diabetes of adults") refers to patients that has a clinical diagnosis of type 2 diabetes, but who is being detected to have autoimmunity towards the pancreatic beta cell.

The term **"HbA1c"** refers to the product of a non-enzymatic glycation of the haemoglobin B chain. Its determination is well known to one skilled in the art. In monitoring the treatment of diabetes mellitus the HbA1c value is of exceptional importance. As its production depends essentially on the blood sugar level and the life of the erythrocytes, the HbA1c in the sense of a "blood sugar memory" reflects the average blood sugar levels of the preceding 4-6 weeks. Diabetic patients whose HbA1c value is consistently well adjusted by intensive diabetes treatment (i.e. < 6.5 % of the total haemoglobin in the sample), are significantly better protected against diabetic microangiopathy. For example, metformin on its own achieves an average improvement in the HbA1c value in the diabetic of the order of 1.0 - 1.5 %. This reduction of the HbA1C value is not sufficient in all diabetics to achieve the desired target range of < 6.5 % and preferably < 6 % HbA1c.

The term **"insufficient glycemic control"** or "inadequate glycemic control" in the scope of the present invention means a condition wherein patients show HbA1c values above 6.5 %, in particular above 7.0 %, even more preferably above 7.5 %, especially above 8 %.

The **"metabolic syndrome",** also called "syndrome X" (when used in the context of a metabolic disorder), also called the "dysmetabolic syndrome" is a syndrome complex with the cardinal feature being insulin resistance (Laaksonen DE, et al. Am J Epidemiol 2002;156:1070-7). According to the ATP III/NCEP guidelines (Executive Summary of the Third Report of the National Cholesterol Education Program (NCEP) Expert Panel on Detection, Evaluation, and Treatment of High Blood Cholesterol in Adults (Adult Treatment Panel III) JAMA: Journal of the American Medical Association (2001) 285:2486-2497), diagnosis of the metabolic syndrome is made when three or more of the following risk factors are present:

1. Abdominal obesity, defined as waist circumference > 40 inches or 102 cm in men, and > 35 inches or 94 cm in women; or with regard to a Japanese ethnicity or Japanese patients defined as waist circumference ≥ 85 cm in men and ≥ 90 cm in women; or otherwise population specific;
2. Triglycerides: ≥ 150 mg/dL
3. HDL-cholesterol < 40 mg/dL in men and <50 in women
4. Blood pressure ≥ 130/85 mm Hg (SBP ≥ 130 or DBP ≥ 85)
5. Fasting blood glucose ≥ 100 mg/dL

The NCEP definitions have been validated (Laaksonen DE, et al. Am J Epidemiol. (2002) 156:1070-7). Triglycerides and HDL cholesterol in the blood can also be determined by standard methods in medical analysis and are described for example in Thomas L (Editor): "Labor und Diagnose", TH-Books Verlagsgesellschaft mbH, Frankfurt/Main, 2000.

According to a commonly used definition, **hypertension** is diagnosed if the systolic blood pressure (SBP) exceeds a value of 140 mm Hg and diastolic blood pressure (DBP) exceeds a value of 90 mm Hg. If a patient is suffering from manifest diabetes it is currently recommended that the systolic blood pressure be reduced to a level below 130 mm Hg and the diastolic blood pressure be lowered to below 80 mm Hg.

The definitions of **NODAT** (new onset diabetes after transplantation) and **PTMS** (post-transplant metabolic syndrome) follow closely that of the American Diabetes Association diagnostic criteria for type 2 diabetes, and that of the International Diabetes Federation (IDF) and the American Heart Association/National Heart, Lung, and Blood Institute, for the metabolic syndrome. NODAT and/or PTMS are associated with an increased risk of micro- and macrovascular disease and events, graft rejection, infection, and death. A number of predictors have been identified as potential risk factors related to NODAT and/or PTMS including a higher age at transplant, male gender, the pre-transplant body mass

index, pre-transplant diabetes, and immunosuppression.

The term **"gestational diabetes"** (diabetes of pregnancy) denotes a form of the diabetes which develops during pregnancy and usually ceases again immediately after the birth. Gestational diabetes is diagnosed by a screening test which often is carried out between the 24th and 28th weeks of pregnancy, but could be conducted at any time during pregnancy, in particular if previous gestational diabetes has been diagnosed. It is usually a simple test in which the blood sugar level is measured e.g., one hour after the administration of 50 g of glucose solution. If this 1 h level is above 140 mg/dl, gestational diabetes is suspected. Final confirmation may be obtained by a standard glucose tolerance test, for example with 75 g of glucose; which also serve as a diagnostic test in the absence of the 50 g challenge.

The term **"hyperuricemia"** denotes a condition of high serum total urate levels. In human blood, uric acid concentrations between 3.6 mg/dL (ca. 214 μmol/L) and 8.3 mg/dL (ca. 494 μmol/L) are considered normal by the American Medical Association. High serum total urate levels, or hyperuricemia, are often associated with several maladies. For example, high serum total urate levels can lead to a type of arthritis in the joints kown as gout. Gout is a condition created by a build up of monosodium urate or uric acid crystals on the articular cartilage of joints, tendons and surrounding tissues due to elevated concentrations of total urate levels in the blood stream. The build up of urate or uric acid on these tissues provokes an inflammatory reaction of these tissues. Saturation levels of uric acid in urine may result in kidney stone formation when the uric acid or urate crystallizes in the kidney. Additionally, high serum total urate levels are often associated with the so-called metabolic syndrome, including cardiovascular disease and hypertension.

The term **"SGLT2 inhibitor"** in the scope of the present invention relates to compounds, in particular to glucopyranosyl-derivatives, i.e. compounds having a glucopyranosyl-moiety, which show an inhibitory effect on the sodium-glucose transporter 2 (SGLT2), in particular the human SGLT2 (hSGLT2). The inhibitory effect on hSGLT2 measured as IC50 is preferably below 1000 nM, even more preferably below 100 nM, most preferably below 50 nM. The inhibitory effect on hSGLT2 can be determined by methods known in the literature, in particular as described in the application WO 2005/092877 or WO 2007/093610 (pages 23/24). The term "SGLT2 inhibitor" also comprises any pharmaceutically acceptable salts thereof, hydrates and solvates thereof, including the respective crystalline forms.

The terms **"treatment"** and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy.

The terms **"prophylactically treating",** "preventivally treating"and "preventing" are used interchangeably and comprise a treatment of patients at risk to develop a condition mentioned hereinbefore, thus reducing said risk.

The term **"tablet"** comprises tablets without a coating and tablets with one or more coatings. Furthermore the "term" tablet comprises tablets having one, two, three or even more layers and press-coated tablets, wherein each of the beforementioned types of tablets may be without or with one or more coatings. The term "tablet" also comprises mini, melt, chewable, effervescent and orally disintegrating tablets.

The terms **"pharmacopoe"** and **"pharmacopoeias"** refer to standard pharmacopoeias such as the "USP 31-NF 26 through Second Supplement" (United States Pharmacopeial Convention) or the "European Pharmacopoeia 6.3" (European Directorate for the Quality of Medicines and Health Care, 2000-2009).

## Brief Description of the Figures

Figure 1 shows an X-ray powder diffractogram of the crystalline form (I.9X) of the compound (1.9). Figure 2 shows the thermoanalysis and determination of the melting point via DSC of the crystalline form (I9.X) of the compound (1.9).

## Detailed Description

The present invention provides a pharmaceutical composition for use in a method for preventing, slowing the progression of, delaying or treating renal hyperfiltrative injury in a patient with type 1 diabetes mellitus. The present invention further provides a pharmaceutical composition for use in a method for preventing, slowing the progression of, delaying or treating renal hyperfiltration in a patient diagnosed with type 1 diabetes mellitus. The pharmaceutical compositions of the present invention comprise the SGLT-2 inhibitor 1-chloro-4-(β-D-glucopyranos-1-yl)-2-[4-((*S*)-tetrahydrofuran-3-yloxy)-benzyl]-benzene.

SGLT2 inhibitors represent a novel class of agents that are being developed for the treatment or improvement in glycemic control in patients with type 2 diabetes. Glucopyranosyl-substituted benzene derivatives are described in the prior art as SGLT2 inhibitors, for example in WO 01/27128, WO 03/099836, WO 2005/092877, WO 2006/034489, WO 2006/064033, WO 2006/117359, WO 2006/117360, WO 2007/025943, WO 2007/028814, WO 2007/031548, WO 2007/093610, WO 2007/128749, WO 2008/049923, WO 2008/055870, WO 2008/055940. The glucopyranosyl-substituted benzene derivatives are proposed as inducers of urinary sugar excretion and as medicaments in the treatment of diabetes.

Renal filtration and reuptake of glucose contributes, among other mechanisms, to the steady state plasma glucose concentration and can therefore serve as an antidiabetic target. Reuptake of filtered glucose across epithelial cells of the kidney proceeds via sodium-dependent glucose cotransporters (SGLTs) located in the brush-border membranes in the tubuli along the sodium gradient. There are at least 3 SGLT isoforms that differ in their expression pattern as well as in their physico-chemical properties. SGLT2 is almost exclusively expressed in the kidney, whereas SGLT1 is expressed additionally in other tissues like intestine, colon, skeletal and cardiac muscle. SGLT3 has been found to be a glucose sensor in interstitial cells of the intestine without any transport function. Potentially, other related, but not yet characterized genes, may contribute further to renal glucose reuptake. Under normoglycemia, glucose is completely reabsorbed by SGLTs in the kidney, whereas the reuptake capacity of the kidney is saturated at glucose concentrations higher than 10mM, resulting in glucosuria ("diabetes mellitus"). This threshold concentration can be decreased by SGLT2-inhibition. It has been shown in experiments with the SGLT inhibitor phlorizin that SGLT-inhibition will partially inhibit the reuptake of glucose from the glomerular filtrate into the blood leading to a decrease in blood glucose concentrations and to glucosuria.

In one aspect, a patient in the context of the present invention is an individual showing renal hyperfiltration or at risk of developing renal hyperfiltration. Such patient is an individual diagnosed or showing type 1 diabetes mellitus (see for example Melsom et al. Diabetes Care 2011; DOI: 10.2337/dc11-0235). Such patient is for example an individual diagnosed or showing type 1 diabetes mellitus, MODY, LADA, pre-diabetes, morbid obesity, congenital or acquired obstructive uro/nephropathy, progressive chronic kidney disease (CKD) and/or acute renal failure (ARF). Such patient is also for example a renal transplant recipient, a renal transplant donors, or an unilateral total or partial nephrectomized patient.

In another aspect, a patient in the context of the present invention is an individual having glomerular filtration rate (GFR) equal to or above 125 ml/min/1.73 $m^2$. In a further aspect, a patient in the context of the present invention is an individual having a GFR equal to or above 140 ml/min/1.73 $m^2$. The GFR of the individual is measured by a method known in the art or as described herein.

In one aspect, the patient:

(1) is an individual diagnosed of one or more of the conditions selected from the group consisting of overweight, obesity, visceral obesity and abdominal obesity; or

(2) is an individual who shows one, two or more of the following conditions:

- (a) a fasting blood glucose or serum glucose concentration greater than 100 mg/dL, in particular greater than 125 mg/dL;
- (b) a postprandial plasma glucose equal to or greater than 140 mg/dL;
- (c) an HbA1c value equal to or greater than 6.0 %, in particular equal to or greater than 6.5%, in particular equal to or greater than 8.0 %;

(3) is an individual wherein one, two, three or more of the following conditions are present:

- (a) obesity, visceral obesity and/or abdominal obesity,
- (b) triglyceride blood level ≥ 150 mg/dL,
- (c) HDL-cholesterol blood level < 40 mg/dL in female patients and < 50 mg/dL in male patients,
- (d) a systolic blood pressure ≥ 130 mm Hg and a diastolic blood pressure ≥ 85 mm Hg,
- (e) a fasting blood glucose level ≥ 100 mg/dL; or

(4) is an individual with morbid obesity.

The SGLT2 inhibitor is the glucopyranosyl-substituted benzene derivative of the formula (1.9)

| | |
|---|---|
| (I.9) | 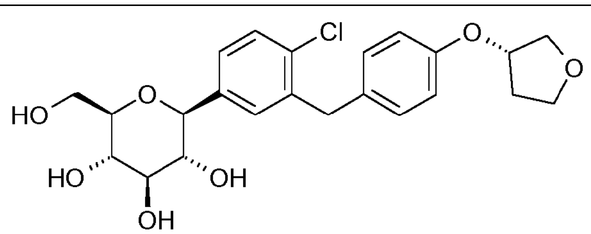 |

(continued)

| | |
|---|---|
| | 1-chloro-4-(β-D-glucopyranos-1-yl)-2-[4-((*S*)-tetrahydrofuran-3-yloxy)-benzyl]-benzene. |

The compound of the formula (1.9) and methods of its synthesis are described for example in the following patent applications: WO 2005/092877, WO 2006/117359, WO 2006/120208, WO 2011/039107, and WO 2011/039108.

According to this invention, it is to be understood that the definition of the above listed glucopyranosyl-substituted benzene derivative of the formula (1.9) also comprises its hydrates, solvates and polymorphic forms thereof, and prodrugs thereof. With regard to the compound (1.9) an advantageous crystalline form is described in the international patent applciation WO 2006/117359 and WO 2011/039107. The crystalline form possess good solubility properties which enable a good bioavailability of the SGLT2 inhibitor. Furthermore, the crystalline form is physico-chemically stable and thus provide a good shelf-life stability of the pharmaceutical composition.

A preferred crystalline form (I.9X) of the compound (1.9) can be characterized by an X-ray powder diffraction pattern that comprises peaks at 18.84, 20.36 and 25.21 degrees 2Θ (±0.1 degrees 2Θ), wherein said X-ray powder diffraction pattern (XRPD) is made using $CuK_{\alpha 1}$ radiation.

In particular said X-ray powder diffraction pattern comprises peaks at 14.69, 18.84, 19.16, 19.50, 20.36 and 25.21 degrees 2Θ (±0.1 degrees 2Θ), wherein said X-ray powder diffraction pattern is made using $CuK_{\alpha 1}$ radiation.

In particular said X-ray powder diffraction pattern comprises peaks at 14.69, 17.95, 18.43, 18.84, 19.16, 19.50, 20.36, 22.71, 23.44, 24.81, 25.21 and 25.65 degrees 2Θ (±0.1 degrees 2Θ), wherein said X-ray powder diffraction pattern is made using $CuK_{\alpha 1}$ radiation.

More specifically, the crystalline form (I.9X) is characterised by an X-ray powder diffraction pattern, made using $CuK_{\alpha 1}$ radiation, which comprises peaks at degrees 2Θ (±0.1 degrees 2Θ) as contained in Table 1.

**Table 1:** X-ray powder diffraction pattern of the crystalline form (I.9X) (only peaks up to 30° in 2 Θ are listed):

| 2 Θ [°] | d-value [Å] | Intensity $I/I_0$ [%] |
|---|---|---|
| 4.46 | 19.80 | 8 |
| 9.83 | 8.99 | 4 |
| 11.68 | 7.57 | 4 |
| 13.35 | 6.63 | 14 |
| 14.69 | 6.03 | 42 |
| 15.73 | 5.63 | 16 |
| 16.20 | 5.47 | 8 |
| 17.95 | 4.94 | 30 |
| 18.31 | 4.84 | 22 |
| 18.43 | 4.81 | 23 |
| 18.84 | 4.71 | 100 |
| 19.16 | 4.63 | 42 |
| 19.50 | 4.55 | 31 |
| 20.36 | 4.36 | 74 |
| 20.55 | 4.32 | 13 |
| 21.18 | 4.19 | 11 |
| 21.46 | 4.14 | 13 |
| 22.09 | 4.02 | 19 |
| 22.22 | 4.00 | 4 |
| 22.71 | 3.91 | 28 |
| 23.44 | 3.79 | 27 |
| 23.72 | 3.75 | 3 |

(continued)

| 2 Θ [°] | d-value [Å] | Intensity $I/I_0$ [%] |
|---|---|---|
| 24.09 | 3.69 | 3 |
| 24.33 | 3.66 | 7 |
| 24.81 | 3.59 | 24 |
| 25.21 | 3.53 | 46 |
| 25.65 | 3.47 | 23 |
| 26.40 | 3.37 | 2 |
| 26.85 | 3.32 | 8 |
| 27.26 | 3.27 | 17 |
| 27.89 | 3.20 | 2 |
| 28.24 | 3.16 | 3 |
| 29.01 | 3.08 | 4 |
| 29.41 | 3.03 | 18 |

Even more specifically, the crystalline form (I.9X) is characterised by an X-ray powder diffraction pattern, made using $CuK_{\alpha 1}$ radiation, which comprises peaks at degrees 2Θ (±0.1 degrees 2Θ) as shown in Figure 1.

Furthermore the crystalline form (I.9X) is characterised by a melting point of about 149°C ± 3°C (determined via DSC; evaluated as onset-temperature; heating rate 10 K/min).The obtained DSC curve is shown in Figure 2.

The X-ray powder diffraction patterns are recorded, within the scope of the present invention, using a STOE - STADI P-diffractometer in transmission mode fitted with a location-sensitive detector □(OED) and a Cu-anode as X-ray source (CuKα1 radiation,□ λ = 1,54056 Å, 40kV, 40mA). In the Table 1 above the values "2Θ [°]" denote the angle of diffraction in degrees and the values "d [Å]" denote the specified distances in Å between the lattice planes. The intensity shown in the Figure 1 is given in units of cps (counts per second).

In order to allow for experimental error, the above described 2Θ values should be considered accurate to ± 0.1 degrees 2Θ, in particular ± 0.05 degrees 2Θ. That is to say, when assessing whether a given sample of crystals of the compound (1.9) is the crystalline form in accordance with the invention, a 2Θ value which is experimentally observed for the sample should be considered identical with a characteristic value described above if it falls within ± 0.1 degrees 2Θ of the characteristic value, in particular if it falls within ± 0.05 degrees 2Θ of the characteristic value.

The melting point is determined by DSC (Differential Scanning Calorimetry) using a DSC 821 (Mettler Toledo).

In one embodiment, a pharmaceutical composition or dosage form according to the present invention comprises the compound (1.9), wherein at least 50 % by weight of the compound (1.9) is in the form of its crystalline form (I.9X) as defined hereinbefore. Preferably in said composition or dosage form at least 80 % by weight, more preferably at least 90 % by weight of the compound (1.9) is in the form of its crystalline form (I.9X) as defined hereinbefore.

Regarding the active pharmaceutical ingredients it can be found that the dissolution properties of the pharmaceutical composition and dosage form is affected *inter alia* by the particle size and particle size distribution of the respective active pharmaceutical ingredient. In the pharmaceutical composition and pharmaceutical dosage form according to the invention the active pharmaceutical ingredients preferably have a particle size distribution such that at least 90 % of the respective active pharmaceutical ingredient particles, with regard to the distribution by volume, has a particle size smaller than 200 μm, i.e. X90 < 200 μm.

In particular, with regard to the glucopyranosyl-substituted benzene derivative of the formula (I), in particular the compound (1.9) or its crystalline form (I.9X), it was found that the particle size influence the manufacturability, in particular that too small particles influence the manufacturability by sticking or filming. On the other hand too large particles negatively affect the dissolution properties of the pharmaceutical composition and dosage form and thus the bioavailability. In the following preferred ranges of the particle size distribution are described.

Therefore, in one aspect, in the pharmaceutical composition and pharmaceutical dosage form according to the invention the glucopyranosyl-substituted benzene derivative of the (1.9), preferably its crystalline form (I9.X), preferably has a particle size distribution (by volume) such that at least 90 % of the respective active pharmaceutical ingredient has a particle size smaller than 200 μm, i.e. X90 < 200 μm, preferably X90 ≤ 150 μm. More preferably the particle size distribution is such that X90 ≤ 100 μm, even more preferably X90 ≤ 90 μm. In addition the particle size distribution is preferably such that X90 ≥ 1 μm, more preferably X90 ≥ 5 μm, even more preferably X90 ≥ 10 μm. Therefore preferred

particle size distributions are such that 1 μm ≤ X90 < 200 μm, particularly 1 μm ≤ X90 ≤ 150 μm, more preferably 5 μm ≤ X90 ≤ 150 μm, even more preferably 5 μm ≤ X90 ≤ 100 μm, even more preferably 10 μm ≤ X90 ≤ 100 μm. A preferred example X90 ≤ 75 μm. Another preferred example is 20 μm ≤ X90 ≤ 50 μm.

Furthermore in the pharmaceutical composition and pharmaceutical dosage form according to the invention the glucopyranosyl-substituted benzene derivative of the formula (1.9), preferably its crystalline form (I9.X), preferably has a particle size distribution (by volume) such that X50 ≤ 90 μm, more preferably X50 ≤ 75 μm, even more preferably X50 ≤ 50 μm, most preferably X50 ≤ 40 μm. In addition the particle size distribution is preferably such that X50 ≥ 1 μm, more preferably X50 ≥ 5 μm, even more preferably X50 ≥ 8 μm. Therefore preferred particle size distributions are such that 1 μm ≤ X50 ≤ 90 μm, particularly 1 μm ≤ X50 ≤ 75 μm, more preferably 5 μm ≤ X50 ≤ 75 μm, even more preferably 5 μm ≤ X50 ≤ 50 μm. A preferred example is 8 μm ≤ X50 ≤ 40 μm.

Furthermore in the pharmaceutical composition and pharmaceutical dosage form according to the invention the glucopyranosyl-substituted benzene derivative of the formula (1.9), preferably its crystalline form (I9.X), preferably has a particle size distribution (by volume) such that X10 ≥ 0.1 μm, more preferably X10 ≥ 0.5 μm, even more preferably X10 ≥ 1 μm, in particular X10 ≥ 2 μm.

Therefore a pharmaceutical composition or pharmaceutical dosage form according to this invention may preferably be characterized by the above specified particle size distributions X90, X50 and/or X10 or one of the following embodiments:

| Embodiment | Glucopyranosyl-substituted benzene derivative (1.9) |
|---|---|
| 1 | X90 < 200 μm |
| 2 | 1 μm ≤ X90 ≤ 150 μm |
| 3 | 5 μm ≤ X90 ≤ 150 μm |
| 4 | 10 μm ≤ X90 ≤ 100 μm |
| 5 | X90 ≤ 150 μm<br>1 μm ≤ X50 ≤ 75 μm |
| 6 | X90 ≤ 150 μm<br>5 μm ≤ X50 ≤ 50 μm |
| 7 | X90 ≤ 150 μm<br>1 μm ≤ X50 ≤ 75 μm<br>X10 ≥ 0.1 μm |
| 8 | X90 ≤ 150 μm<br>5 μm ≤ X50 ≤ 50 μm<br>X10 ≥ 0.5 μm |

The value X90 refers to the 90% value of the volume distribution measured using a laser diffractometer. In other words, for the purposes of the present invention, the X90 value denotes the particle size below which 90% of the quantity of particles is found based on the volume distribution. Analogously the value X50 refers to the 50% value (median) of the volume distribution measured using a laser diffractometer. In other words, for the purposes of the present invention, the X50 value denotes the particle size below which 50% of the quantity of particles is found based on the volume distribution. Analogously the value X10 refers to the 10% value of the volume distribution measured using a laser diffractometer. In other words, for the purposes of the present invention, the X10 value denotes the particle size below which 10% of the quantity of particles is found based on the volume distribution.

Preferably all X90, X50, X10 values hereinbefore and hereinafter are by volume and determined by laser-diffraction method, in particular low angle laser light scattering, i.e. Fraunhofer diffraction. A preferred test is described in the experimental section. The laser diffraction method is sensitive to the volume of a particle and provides a volume-average particle size, which is equivalent to the weight-average particle size if the density is constant. The skilled artesian knows that the results of the particle size distribution determination by one technique can be correlated with that from another technique, for example on an empirical basis by routine experimentation. Alternatively the particle size distribution in the pharmaceutical composition or dosage form can be determined by microscopy, in particular electron microscopy or scanning electron microscopy.

In the following the suitable excipients and carriers in the pharmaceutical compositions according to the invention are described in further detail.

A pharmaceutical composition according to the invention typically comprises one or more diluents, one or more

disintegrants and optionally one or more binders. Some of the excipients may have two or more functions at the same time, e.g. act as a filler and a binder.

Suitable diluents according to the invention are for example, lactose, in particular lactose monohydrate, cellulose and derivatives, such as powdered cellulose, microcrystalline or silicified microcrystalline cellulose, cellulose acetate, starches and derivatives such as pregelatinized starch, corn starch, wheat starch, rice starch, potato starch, sterilizable maize, sodium chloride, calcium carbonate, calcium phosphate, particularly dibasic calcium phosphate, calcium sulphate, dicalcium or tricalcium phosphate, magnesium carbonate, magnesium oxide, sugars and derivatives such as confectioner's sugar, fructose, sucrose, dextrates, dextrin, D-sorbitol sulfobutylether β-cyclodextrin, dextrose, polydextrose, trehalose, maltose, maltitol, mannitol, maltodextrin, sorbitol, inulin, xylitol, erythritol, isomalt, kaolin and lactitol. Preferred diluents are lactose monohydrate and microcrystalline cellulose.

Suitable disintegrants according to the invention are for example powdered cellulose, crospovidone, croscarmellose sodium, docusate sodium, low-substituted hydroxypropyl cellulose, magnesium aluminum silicate, microcrystalline cellulose, polacrilin potassium, sodium starch glycolate, starch, particularly pregelatinized starch and corn starch. A preferred disintegrant is croscarmellose sodium.

Any binder usually employed in pharmaceutical compositions may be used in the context of the instant invention. Binders are for example naturally occurring or partially or totally synthetic polymers selected from acacia, agar, alginic acid, carbomers, carmellose sodium, carrageenan, cellulose acetate phthalate, ceratonia, chitosan, confectionar's sugar, copovidone, povidone, cottonseed oil, dextrate, dextrin, dextrose, polydextrose, maltodextrin, maltose, cellulose and derivatives thereof such as microcrystalline cellulose, methylcellulose, ethylcellulose, hydroxyethyl cellulose, hydroxyethyl methylcellulose, hydroxypropyl celluloses, carboxymethylcelluloses, hypromelloses (cellulose hydroxypropyl methyl ether), starch and derivatives thereof, such as pregelatinized starch, hydroxypropylstarch, corn starch, gelatin, glyceryl behenate, tragacanth, guar gum, hydrogenated vegetable oils, inulin, poloxamer, polycarbophils, polyethylene oxide, polyvinylpyrrolidone, copolymers of N-vinylpyrrolidone and vinyl acetate, polymethacrylates, polyethylene glycols, alginates such as sodium alginate, gelatin, sucrose, sunflower oil, zein as well as derivatives and mixtures thereof. Preferred binders are microcrystalline cellulose and hydroxypropyl cellulose.

The pharmaceutical composition according to the present invention may also comprise one or more lubricants. Suitable lubricants according to the invention are stearic acid as well as salts thereof including talc, sodium stearate, calcium stearate, zinc stearate, magnesium stearate, sodium stearyl fumarate, glyceryl monostearate, particularly magnesium stearate, polyethylene glycols, in particular polyethylene glycol with a molecular weight in a range from about 4400 to about 9000, hydrogenated castor oil, fatty acid, for example fumaric acid, and salts of fatty acids, in particular the calcium, magnesium, sodium or pottasium salts thereof, for example calcium behenate, calcium stearate, sodium stearyl fumarate or magnesium stearate (for example (e.g. HyQual®, Mallinckrodt), glycerides such as glyceryl behenate (Compritol® 888), Dynasan® 118 or Boeson® VP.

The pharmaceutical composition according to the present invention may also comprise one or more glidants. Suitable glidants according to the invention are silicon dioxide, particularly colloidal silicon dioxide (e.g. Aerosil®, Cab-O-Sil®), stearic acid as well as salts thereof including sodium stearate, calcium stearate, zinc stearate, magnesium stearate, magnesium silicate, calcium silicate, magnesium trisilicate and talc. Preferred glidants are colloidal silicon dioxide and talc.

In another embodiment, a pharmaceutical composition according to the instant invention comprises

| | Amount (% by weight) |
|---|---|
| Active ingredient | 0.5 - 25 |
| One or more diluents | 65 - 93 |
| One or more binders | 1 - 5 |
| One or more disintegrants | 1 - 4 |
| Optionally additional additives | ad 100 % |

The active ingredient is the compound of the formula (1.9) or its crystalline form (I.9X).

In another embodiment, a pharmaceutical composition according to the instant invention comprises

| | Amount (% by weight) |
|---|---|
| Active ingredient | 0.5 - 25 |
| One or more diluents | 65 - 90 |

(continued)

| | Amount (% by weight) |
|---|---|
| One or more binders | 1 - 5 |
| One or more disintegrants | 1 - 3 |
| Optionally additional additives | ad 100 % |

The active ingredient is the compound of the formula (1.9) or its crystalline form (I.9X).

In another embodiment, a pharmaceutical composition according to the instant invention comprises

| | Amount (% by weight) |
|---|---|
| Active ingredient | 0.5 - 25 |
| Lactose monohydrate | 28 - 70 |
| Microcrystalline cellulose | 20 - 50 |
| Hydroxypropyl cellulose | 1 - 5 |
| Croscarmellose sodium | 1 - 4 |
| Optionally additional additives | ad 100 % |

The active ingredient is a compound of the formula (1.9) or its crystalline form (I.9X).

In another embodiment, a pharmaceutical composition according to the instant invention comprises

| | Amount (% by weight) |
|---|---|
| Active ingredient | 0.5 - 25 |
| Lactose monohydrate | 35 - 90 |
| Microcrystalline cellulose | 0 - 30 |
| Hydroxypropyl cellulose | 1 - 5 |
| Croscarmellose sodium | 1 - 3 |
| Additional additives | ad 100 % |

The active ingredient is a compound of the formula (1.9) or its crystalline form (I.9X).

In one embodiment, the ratio of said disintegrant to said binder in a pharmaceutical composition of the present invention is between 1.5:3.5 and 1:1.

In one embodiment, the active ingredient represents 25% or less of the weight of the pharmaceutical composition. Preferably, the active ingredient represents 0.5% to 25% of the weight of the pharmaceutical composition. More preferably, the active ingredient represents 1.0% to 20% of the weight of the pharmaceutical composition. Even more preferably, the active ingredient represents 2.0% to 15% of the weight of the pharmaceutical composition.

In the following, preferred ranges of the amount of the glucopyranosyl-substituted benzene derivative to be employed in the pharmaceutical dosage form according to this invention are described. These ranges refer to the amounts to be administered per day with respect to an adult patient, in particular to a human being, for example of approximately 70 kg body weight, and can be adapted accordingly with regard to an administration 1, 2, 3, 4 or more times daily and with regard to other routes of administration and with regard to the age of the patient. The ranges of the dosage and amounts are calculated for the active ingredient.

A preferred amount of the glucopyranosyl-substituted benzene derivative (1.9) or its crystalline form (I.9X) is in a range from 0.5 to 100 mg, preferably from 0.5 to 50 mg, even more preferably from 1 to 25 mg, even more preferably 5 to 25 mg, particularly 10 to 25 mg. Preferred dosages of the glucopyranosyl-substituted benzene derivative are for example 1 mg, 2 mg, 2.5 mg, 5 mg, 7.5 mg, 10 mg, 12.5 mg, 15 mg, 20 mg, 25 mg and 50 mg, in particular 10 mg and 25 mg.

A pharmaceutical composition according to the present invention may be comprised in a tablet, a capsule or a film-coated tablet,

In one embodiment, a tablet comprising a pharmaceutical composition according to the present invention comprises a lubricant, such as magnesium stearate. Such lubricant may be present in a concentration of 0.25 - 2 % in said tablet.

In one embodiment, a tablet comprising a pharmaceutical composition according to the present invention comprises a glidant, such as colloidal silicon dioxide. Such glidant may be present in a concentration of 0.25 - 2 % in said tablet.

A tablet according to the invention may be film-coated. Typically a film coat represents 2-5% by weight of the total composition and comprises preferably a film-forming agent, a plasticizer, a glidant and optionally one or more pigments. An exemplary coat composition may comprise hydroxypropylmethyl-cellulose (HPMC), polyethylene glycol (PEG), talc, titanium dioxide and optionally iron oxide, including iron oxide red and/or yellow.

In one embodiment, the pharmaceutical dosage form according to the invention has dissolution properties such that after 45 minutes at least 75 %, preferably at least 90 % by weight of the pharmaceutical active ingredient is dissolved. In another embodiment after 30 minutes at least 75 %, preferably at least 90 % by weight of the pharmaceutical active ingredien is dissolved. In another embodiment after 15 minutes at least 75 %, preferably at least 90 % by weight of the pharmaceutical active ingredient is dissolved. The dissolution properties can be determined in a standard dissolution test, for example as described in pharmacopoeias, such as the USP31-NF26 S2, chapter 711 (dissolution).

In one embodiment, the pharmaceutical dosage form according to the invention has disintegration properties such that within 40 minutes, alternatively within 30 minutes, preferably within 20 minutes, more preferably within 15 minutes the pharmaceutical dosage form is disintegrated. The disintegration properties can be determined in a standard disintegration test, for example as described in pharmacopoeias, such as the USP31-NF26 S2, chapter 701 (disintegration).

In one embodiment, the pharmaceutical dosage form according to the invention has a high content uniformity, preferably within a range from 85 to 115 %, more preferably from 90 to 110 %, even more preferably from 95 to 105 % by weight with regard to the pharmaceutical ingredient. The content uniformity can be determined in a standard test using for example randomly 10 selected pharmaceutical dosage forms, for example as described in pharmacopoeias.

A dosage form according to this invention, such as a tablet, capsule or film-coated tablet, may be prepared by methods well-known to the one skilled in the art.

Suitable methods of manufacturing a tablet include compression of the pharmaceutical composition in the form of a powder, i.e. direct compression, or compression of the pharmaceutical composition in the form of granules, and if needed with additional excipients.

Granules of the pharmaceutical composition according to the invention may be prepared by methods well-known to the one skilled in the art. Preferred methods for the granulation of the active ingredients together with the excipients include wet granulation, for example high shear wet granulation and fluidized bed wet granulation, dry granulation, also called roller compaction.

In the wet granulation process the granulation liquid are the solvent alone or a preparation of one or more binders in a solvent or mixture of solvents. Suitable binders are described hereinbefore. Examples are hypromellose, hydroxypropyl cellulose, povidone and copovidone. Suitable solvents are for example purified water, ethanol, methanol, isopropanol, acetone, preferably purified water, including mixtures thereof. The solvent is a volatile component, which does not remain in the final product. The one or more active ingredients and the other excipients, in particular the one or more diluents and the one or more disintegrants, usually with exception of the lubricant, are premixed and granulated with the granulation liquid, for example using a high shear granulator. The wet granulation step is usually followed by one or more drying and sieving steps. For example a drying oven or a fluid bed dryer can then be used for drying.

The dried granules are sieved through an appropriate sieve. After optional addition of the other excipients, in particular disintegrant, binder, filler and/or glidant, with exception of the lubricant the mixture is blended in a suitable blender, for example a free fall blender, followed by addition of the one or more lubricants, for example magnesium stearate, and final blending in the blender.

An exemplary wet granulation process for making a pharmaceutical composition according to the instant invention comprises the steps of:

(1) Premixing the active ingredient and the main portion of the excipients including the binder in a mixer to obtain a pre-mixture;
(2) granulating the pre-mixture of step (1) by adding the granulation liquid, preferably purified water;
(3) drying the granules of step (2) in a fluidized bed dryer or a drying oven;
(4) optionally dry sieving of the dried granules of step (3);
(5) mixing the dried granules of step (4) with the remaining excipients like filler, binder, disintegrant and/or glidant in a mixer to obtain the main mixture;
(6) mixing the main mixture of step (5) with the lubricnat in a mixer to obtain the final mixture;
(7) tableting the final mixture of step (6) by compressing it on a suitable tablet press to produce tablets cores;

(8) optionally film-coating of the tablet cores of step (7) with a non-functional coat.

The present invention also provides a pharmaceutical composition obtainable by the above process.

An exemplary direct compression process according to the present invention for making a pharmaceutical composition comprises the steps of:

(1) Premixing the active ingredient and the main portion of the excipients in a mixer to obtain a pre-mixture;
(2) optionally dry screening the pre-mixture through a screen in order to segregate cohesive particles and to improve content uniformity;
(3) mixing the pre-mixture of step (1) or (2) in a mixer, optionally by adding remaining excipients to the mixture and continuing mixing;
(4) tableting the final mixture of step (3) by compressing it on a suitable tablet press to produce the tablet cores;
(5) optionally film-coating of the tablet cores of step (4) with a non-functional coat.

The present invention also provides a pharmaceutical composition obtainable by the above process.

An exemplary dry granulation process according to the present invention for making a pharmaceutical composition comprises the steps of:

(1) mixing the active ingredient or a pharmaceutically acceptable salt thereof with either all or a portion of the excipients in a mixer;
(2) compaction of the mixture of step (1) on a suitable roller compactor;
(3) reducing the ribbons obtained during step (2) to small granules by suitable milling or sieving steps;
(4) optionally mixing the granules of step (3) with the remaining excipients in a mixer to obtain the final mixture;
(5) tabletting the granules of step (3) or the final mixture of step (4) by compressing it on a suitable tablet press to produce the tablet cores;
(6) optionally film-coating of the tablet cores of step (5) with a non-functional coat.

In one embodiment, the size of the granules according to the present invention is in the range from 25 to 800 μm, for example from 40 μm to 500 μm. The size of the granules may be measured via sieve analysis, for example with a sonic sifter. In one embodiment, at least 80 %, at least 90 %, or at least 95 % by weight of the granules is in the given range.

When this invention refers to patients requiring treatment or prevention, it relates primarily to treatment and prevention in humans, but the pharmaceutical composition may also be used accordingly in veterinary medicine in mammals. In the scope of this invention adult patients are preferably humans of the age of 18 years or older. Also in the scope of this invention, patients are adolescent humans, i.e. humans of age 6 to 17 years, for example 10 to 17 years, preferably of age 13 to 17 years.

As described hereinbefore by the administration of the pharmaceutical composition according to this invention and in particular in view of the high SGLT2 inhibitory activity of the SGLT2 inhibitor therein, excessive blood glucose is excreted through the urine of the patient, so that no gain in weight or even a reduction in body weight may result. Therefore, a treatment or prophylaxis according to this invention is advantageously suitable in those patients in need of such treatment or prophylaxis who are diagnosed of one or more of the conditions selected from the group consisting of overweight and obesity, in particular class I obesity, class II obesity, class III obesity, morbid obesity, visceral obesity and abdominal obesity. In addition a treatment or prophylaxis according to this invention is advantageously suitable in those patients in which a weight increase should preferably be avoided. The pharmaceutical composition according to the present invention allow a reduction of the HbA1c value to a desired target range, for example < 7 % and preferably < 6.5 %, for a higher number of patients and for a longer time of therapeutic treatment compared with a corresponding monotherapy or a therapy using only two of the combination partners.

The pharmaceutical composition according to this invention and in particular the SGLT2 inhibitor therein exhibits a very good efficacy with regard to glycemic control, in particular in view of a reduction of fasting plasma glucose, postprandial plasma glucose and/or glycosylated hemoglobin (HbA1c). By administering a pharmaceutical composition according to this invention, a reduction of HbA1c equal to or greater than preferably 0.5 %, even more preferably equal to or greater than 1.0 % can be achieved and the reduction is particularly in the range from 1.0 % to 2.0 %.

Furthermore, the pharmaceutical composition according to this invention is advantageously applicable in those patients who show one, two or more of the following conditions:

(a) a fasting blood glucose or serum glucose concentration greater than 100 mg/dL, in particular greater than 125 mg/dL;
(b) a postprandial plasma glucose equal to or greater than 140 mg/dL;
(c) an HbA1c value equal to or greater than 6.0 %, equal to or greater than 6.5 %, equal to or greater than 7.0 %,

equal to or greater than 7.5 %, or equal to or greater than 8.0 %.

In the following preferred ranges of the amount of the SGLT2 inhibitor to be employed in the pharmaceutical composition for use in methods according to this invention are described. These ranges refer to the amounts to be administered per day with respect to an adult patient, in particular to a human being, for example of approximately 70 kg body weight, and can be adapted accordingly with regard to an administration 1, 2, 3, 4 or more times daily and with regard to other routes of administration and with regard to the age of the patient. Within the scope of the present invention, the pharmaceutical composition is preferably administered orally. Other forms of administration are possible and described hereinafter. Preferably the one or more dosage forms comprising the SGLT2 inhibitor is oral or usually well known.

In general, the amount of the SGLT2 inhibitor in the pharmaceutical composition for use in methods according to this invention is preferably the amount usually recommended for a monotherapy using said SGLT2 inhibitor.

The preferred dosage range of the SGLT2 inhibitor is in the range from 0.5 mg to 200 mg, even more preferably from 1 to 100 mg, most preferably from 1 to 50 mg per day. The oral administration is preferred. Therefore, a pharmaceutical composition may comprise the hereinbefore mentioned amounts, in particular from 1 to 50 mg or 1 to 25 mg. Particular dosage strengths (e.g. per tablet or capsule) are for example 1, 2.5, 5, 7.5, 10, 12.5, 15, 20, 25 or 50 mg, in particular 10 mg or 25 mg of the SGLT2 inhibitor of the formula (1.9) or its crystalline form (I.9X). The application of the active ingredient may occur up to three times a day, preferably one or two times a day, most preferably once a day.

The hereinbefore and hereinafter described pharmaceutical compositions for use in methods are employed by administering the pharmaceutical composition comprising the SGLT2 inhibitor (1.9) to the patient. An oral administration is preferred.

In one embodiment of the pharmaceutical compositions according to the present invention the SGLT2 inhibitor, in particular the compound (1.9) is the only active pharmaceutical ingredient administered to the patient. Thus the pharmaceutical compositions for use in methods relate to a mono-therapy.

In another embodiment of the pharmaceutical compositions for use in methods according to the present invention the SGLT2 inhibitor (1.9) is administered to the patient in combination with one or more other active pharmaceutical ingredients. Thus the methods relate to a combination therapy. Preferably the other active pharmaceutical ingredient is a medicament for the treatment of diabetes mellitus, in particular type 1 diabetes mellitus or type 2 diabetes mellitus. Therefore the pharmaceutical compositions for use in methods according to the present invention also relate to a combination therapy with one or more other antidiabetics. The other active pharmaceutical ingredient as a medicament for the treatment of diabetes mellitus is preferably selected from the group consisting of metformin, a DPPIV inhibitor, an insulin, a PPARgamma agonist, a GLP-1 receptor agonist, a glinide, a sulfonylurea or an alpha-glucosidase blocker, including combinations thereof. Specific examples of other pharmaceutical ingredients are metformin, linagliptin, repaglinide, liraglutide, pioglitazone and glimepirid. Combinations of a SGLT2 inhibitor and an antidiabetic active pharmaceutical ingredient are described for example in WO 2008/055940.

The pharmaceutical compositions for use in methods according to the present invention may be of particular advantage in those patients who are pretreated with an antidiabetic medicament and who have a risk to develop hyperfiltration or who are diagnosed of having hyperfiltration. The co-administration of the SGLT2 inhibitor (1.9) in combination with the other antidiabetc may allow to treat hyperfiltration, including to lower the risk of developing hyperfiltration, while lowering the blood-glucose levels through excretion of glucose via the urine and thus having the opportunity to lower the dosage of the other antidiabetic medicament. Further advantage of the co-administration of the SGLT2 inhibitor is that a weight loss and/or a reduction of body fat may be achieved or an increase of weight and/or body fat which would be provoked by the other antidiabetic may be prevented or attenuated. In addition by using the SGLT2 inhibitor the blood pressure may be lowered which is of particular advantage in patients having an elevated risk of high blood pressure or being diagnosed of having a high blood pressure.

The SGLT2 inhibitor (1.9) and the other active pharmaceutical ingredient, in particular an antidiabetic, may be formulated into one pharmaceutical composition for combined administration or may be formulated into separate pharmaceutical compositions for combined or separate administration.

The term metformin includes its salts, such as metformin hydrochloride. Furthermore metformin may be comprised in a pharmaceutical composition for immediate release, for modified release, sustained release or for extended release. Metformin may be administered orally using pharmaceutical compositions, dosages and administration schemes as known in the prior art. A preferred dosage range for metformin is 500 to 2000 mg once or twice daily, for example 500 mg, 850 mg or 1000 mg, preferably once or twice daily. In the pharmaceutical compositions according to the present invention the SGLT2 inhibitor (1.9) and metformin may be administered in combination, for example simultaneously, and when they are administered in alternation, for example successively in separate formulations. Methods for the combined administration of a SGLT2 inhibitor and metformin are described for example in WO 2008/055940. Pharmaceutical compositions comprising a SGLT2 inhibitor, in particular the compound (1.9), and metformin are described in WO 2011/039337. Preferred dosages of the combination compound (I.9)/ metformin are for example 5 mg/ 500 mg, 12.5 mg/ 500 mg, 5 mg/ 850 mg, 12.5 mg/ 850 mg, 5 mg/ 1000 mg and 12.5 mg/ 1000 mg.

The DPPIV inhibitor may be selected from the group consisting of linagliptin, sitagliptin, saxagliptin, vildagliptin, alogliptin, denagliptin, melogliptin, carmegliptin, dutogliptin, tenegliptin, gosogliptin, anagliptin, gemigliptin and bisegliptin. The DPPIV inhibitor may be administered orally using pharmaceutical compositions, dosages and administration schemes as known in the prior art. A preferred DPPIV inhibitor is linagliptin. As linagliptin is primarily excreted by bile and the gut a higher safety in patients with renal conditions or dysfunction is seen. Therefore a combination of the compound (1.9) with linagliptin is of particular interest in the pharmaceutical compositions according to this invention. A preferred dosage range for linagliptin is 0.5 to 20 mg once or twice daily, for example 0.5 mg, 1 mg, 2.5 mg, 5 mg or 10 mg, preferably once daily. In the pharmaceutical compositions for use in methods according to the present invention the SGLT2 inhibitor, in particular the compound (1.9), and the DPPIV inhibitor, for example linagliptin, may be administered in combination, for example simultaneously, and when they are administered in alternation, for example successively in separate formulations. Methods for the combined administration of a SGLT2 inhibitor and a DPPIV inhibitor are described for example in WO 2009/022007. Pharmaceutical compositions comprising a SGLT2 inhibitor, in particular the compound (1.9), and the DPPIV inhibitor, for example linagliptin, are described in WO 2010/092124. Preferred dosages of the combination compound (I.9)/ linagliptin are for example 10 mg/ 5 mg, 12.5 mg/ 5 mg and 25 mg/ 5 mg.

In addition the pharmaceutical compositions for use in methods according to the present invention relate to an administration of the SGLT2 inhibitor (1.9) and the DPPIV inhibitor, for example linagliptin, in combination or alternation with another active pharmaceutical ingredient for the treatment of diabetes mellitus. An example of another active pharmaceutical ingredient is metformin. Combinations of an SGLT2 inhibitor, a DPPIV inhibitor and a third active pharmaceutical ingredient are described in the WO 2010/092125.

The PPARgamma agonist is preferably a thiazolidindione, in particular pioglitazone or rosiglitazone. The term "pioglitazone" as employed herein refers to pioglitazone, including its enantiomers, mixtures thereof and its racemate, or a pharmaceutically acceptable salt thereof such as the hydrochloride salt. The term "rosiglitazone" as employed herein refers to rosiglitazone, including its enantiomers, mixtures thereof and its racemate, or a pharmaceutically acceptable salt thereof such as the maleate salt.

The term "insulin" in the scope of the present invention relates to insulin and insulin analogs being used in the therapy of patients, in particular humans, which includes normal insulin, human insulin, insulin derivatives, zinc insulins and insulin analogues, including formulations thereof with modified release profiles. in particular as used in the therapy of humans. The term "insulin" in the scope of the present invention covers the following types of insulins:

- rapid-acting insulins,
- short-acting insulins,
- intermediate-acting insulins,
- long-acting insulins,

and mixtures thereof, for example mixtures of short- or rapid-acting insulins with long-acting insulins. The term "insulin" in the scope of the present invention covers insulins which are administered to the patient via injection, via infusion, including pumps, via inhalation, via oral, via transdermal or other routes of administration. Examples of insulins are regular insulin or human insulin, NPH insulin, also known as Humulin N, Novolin N, Novolin NPH and isophane insulin, lente insulins, such as Semilente or Monotard, insulin glargine, insulin detemir or insulin degludec, insulin lispro PEGylated, amidated insulin glargine.

The term "GLP-1 receptor agonist" in the scope of the present invention includes, without being limited, exogenous GLP-1 (natural or synthetic), GLP-1 analogues and other substances (whether peptidic or non-peptidic, e.g. small molecules) which promote signalling through the GLP-1 receptor. The exogenous GLP-1 includes natural and synthetic GLP-1, in particular human GLP-1. The GLP-1 analogues include longer acting analogues also which are resistant to or have reduced susceptibility to enzymatic degradation, for example by DPP-4 and/or NEP 24.11. Examples of GLP-1 analogues are selected from the group consisting of exenatide (exendin-4); exenatide LAR (long acting release formulation of exenatide); liraglutide; taspoglutide; semaglutide; albiglutide; lixisenatide; dulaglutide; and the PEGylated GLP-1 compound comprising the amino acid sequence according to the claim 1 of WO 2006/124529 (the disclosure of which is incorporated herein) and the GLP-1 derivative comprising the amino acid sequence according to SEQ ID NO:21 as disclosed in the WO 2009/020802 (the disclosure of which is incorporated herein).

Combinations of an insulin and a GLP-1 receptor agonist, in particular a GLP-1 analog, may be used in combination with the SGLT-2 inhibitor (1.9) in the pharmaceutical compositions for use in methods according to this invention also.

Examples of glinides are repaglinide and nateglinide. Examples of sulfonylureas are glibenclamide, tolbutamide, glimepiride, glipizide, glyburide, gliclazide. Examples of alpha-glucosidase blockers are miglitol, acarbose and voglibose.

According to a first embodiment a manufacture comprises (a) a pharmaceutical composition comprising the SGLT2 inhibitor according to the present invention and (b) a label or package insert which comprises instructions that the medicament is to be administered.

The desired dose of the pharmaceutical composition according to this invention may conveniently be presented in a once daily or as divided dose administered at appropriate intervals, for example as two, three or more doses per day.

The pharmaceutical composition may be formulated for oral, rectal, nasal, topical (including buccal and sublingual), transdermal, vaginal or parenteral (including intramuscular, subcutaneous and intravenous) administration in liquid or solid form or in a form suitable for administration by inhalation or insufflation. Oral administration is preferred. The formulations may, where appropriate, be conveniently presented in discrete dosage units and may be prepared by any of the methods well known in the art of pharmacy. All methods include the step of bringing into association the active ingredient with one or more pharmaceutically acceptable carriers, like liquid carriers or finely divided solid carriers or both, and then, if necessary, shaping the product into the desired formulation.

The pharmaceutical composition may be formulated in the form of tablets, granules, fine granules, powders, capsules, caplets, soft capsules, pills, oral solutions, syrups, dry syrups, chewable tablets, troches, effervescent tablets, drops, suspension, fast dissolving tablets, oral fast-dispersing tablets, etc..

The pharmaceutical composition and the dosage forms preferably comprises one or more pharmaceutical acceptable carriers which must be "acceptable" in the sense of being compatible with the other ingredients of the formulation and not deleterious to the recipient thereof. Examples of pharmaceutically acceptable carriers are known to the one skilled in the art.

Pharmaceutical compositions suitable for oral administration may conveniently be presented as discrete units such as capsules, including soft gelatin capsules, cachets or tablets each containing a predetermined amount of the active ingredient; as a powder or granules; as a solution, a suspension or as an emulsion, for example as syrups, elixirs or self-emulsifying delivery systems (SEDDS). The active ingredients may also be presented as a bolus, electuary or paste. Tablets and capsules for oral administration may contain conventional excipients such as binding agents, fillers, lubricants, disintegrants, or wetting agents. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservatives.

The pharmaceutical composition according to the invention may also be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredients may be in powder form, obtained by aseptic isolation of sterile solid or by lyophilisation from solution, for constitution with a suitable vehicle, e.g. sterile, pyrogen-free water, before use.

Pharmaceutical compositions suitable for rectal administration wherein the carrier is a solid are most preferably presented as unit dose suppositories. Suitable carriers include cocoa butter and other materials commonly used in the art, and the suppositories may be conveniently formed by admixture of the active compound(s) with the softened or melted carrier(s) followed by chilling and shaping in moulds.

The pharmaceutical compositions for use in methods according to this invention show advantageous effects in the treatment and prevention of those diseases and conditions as described hereinbefore. Advantageous effects may be seen for example with respect to efficacy, dosage strength, dosage frequency, pharmacodynamic properties, pharmacokinetic properties, fewer adverse effects, convenience, compliance, etc..

Methods for the manufacture of SGLT2 inhibitor according to this invention and of prodrugs thereof are known to the one skilled in the art. Advantageously, the compounds according to this invention can be prepared using synthetic methods as described in the literature, including patent applications as cited hereinbefore. Preferred methods of manufacture are described in the WO 2006/120208 and WO 2007/031548. With regard to compound (1.9) an advantageous crystalline form is described in the international patent application WO 2006/117359 and WO 2011/039108.

Any of the above mentioned pharmaceutical compositions and methods within the scope of the invention may be tested by animal models known in the art.

Pharmaceutical compositions for use in methods according to this invention can be tested in genetically hyperinsulinemic or diabetic animals like db/db mice, ob/ob mice, Zucker Fatty (fa/fa) rats or Zucker Diabetic Fatty (ZDF) rats. In addition, they can be tested in animals with experimentally induced diabetes like HanWistar or Sprague Dawley rats pretreated with streptozotocin.

## Pharmacological Examples

The following examples show the beneficial effect on glycemic control and hyperfiltration of the pharmaceutical compositions according to the present invention. The SGLT-2 inhibitor is 1-chloro-4-(β-D-glucopyranos-1-yl)-2-[4-((*S*)-tetrahydrofuran-3-yloxy)-benzyl]-benzene, preferably in the crystalline form (I.9X).

**Example 1: Treatment of hyperfiltration**

In clinical studies running for different lengths of time (e.g. 1 day to 24 months) the success of the treatment in patients with hyperfiltration is checked by determining the change in glomerular filtration rate (GFR) after treatment with the SGLT-2 inhibitor under controlled conditions of euglycaemia and hyperglycaemia. Subjects are administered 25 mg of the SGLT-2 inhibitor once daily.
A significant change in GFR in patients with hyperfiltration, during or at the end of the study, compared with the initial value or compared with a placebo group, or a group given a different therapy, proves the efficacy of a pharmaceutical composition according to the invention in the treatment of hyperfiltration. The GFR is determined as described herein.
Additional parameters of renal function may also be measured. These include:

- Change in renal hemodynamic function: Effective Renal Plasma Flow (ERPF), Renal Blood Flow (RBF), Filtration Fraction (FF) and Renal Vascular Resistance (RVR);
- Change in systemic hemodynamic function: Mean Arterial Pressure (MAP) and arterial stiffness;
- Change in circulating levels of mediators involved in Renin-Angiotensin-Aldosterone System (RAAS) activation and markers of sympathetic activity;
- Change in urinary measurements of nitric oxide, prostanoids and albumin excretion.

The parameters are determined using techniques known in the art.

**Examples of Formulations**

The following examples of formulations, which may be obtained analogously to methods known in the art, serve to illustrate the present invention more fully without restricting it to the contents of these examples. The term "active substance" denotes the SGLT-2 inhibitor of the formula (1.9) or its crystalline form (I.9X).
The active pharmaceutical ingredient or active sustance, i.e. the compound (1.9), preferably in the crystalline form (I.9X), is milled with a suitable mill like pin- or jet-mill in order to obtain the desired particle size distribution before manufacturing of the pharmaceutical composition or dosage form.
Examples of typical particle size distribution values X90, X50 and X10 for the preferred active pharmaceutical ingredient according to the invention are shown in the table below.

Typical particle size distribution results

| | Active substance Batch 1 | Active substance Batch 2 |
|---|---|---|
| X10 | 1,8 μm | 1,7 μm |
| X50 | 18,9 μm | 12,1 μm |
| X90 | 45,3 μm | 25,9 μm |

Example 1: Dry ampoule containing 50 mg of active substance per 10 ml

Composition:

| | |
|---|---|
| Active substance | 50.0 mg |
| Mannitol | 50.0 mg |
| water for injections | ad 10.0 ml |

Preparation:

Active substance and mannitol are dissolved in water. After packaging the solution is freeze-dried. To produce the solution ready for use, the product is dissolved in water for injections.

Example 2: Dry ampoule containing 25 mg of active substance per 2 ml

Composition:

| | |
|---|---|
| Active substance | 25.0 mg |
| Mannitol | 100.0 mg |
| water for injections | ad 2.0 ml |

Preparation:

Active substance and mannitol are dissolved in water. After packaging, the solution is freeze-dried. To produce the solution ready for use, the product is dissolved in water for injections.

Example 3: Tablet containing 50 mg of active substance

| | |
|---|---|
| Composition: | |
| (1) Active substance | 50.0 mg |
| (2) Mannitol | 98.0 mg |
| (3) Maize starch | 50.0 mg |
| (4) Polyvinylpyrrolidone | 15.0 mg |
| (5) Magnesium stearate | 2.0 mg |
| | 215.0 mg |

Preparation:

(1), (2) and (3) are mixed together and granulated with an aqueous solution of (4). (5) is added to the dried granulated material. From this mixture tablets are pressed, biplanar, faceted on both sides and with a dividing notch on one side.

Diameter of the tablets: 9 mm.

Example 4: Capsules containing 50 mg of active substance

Composition:

| | |
|---|---|
| (1) Active substance | 50.0 mg |
| (2) Dried maize starch | 58.0 mg |
| (3) Mannitol | 50.0 mg |
| (4) Magnesium stearate | 2.0 mg |
| | 160.0 mg |

Preparation:

(1) is triturated with (3). This trituration is added to the mixture of (2) and (4) with vigorous mixing. This powder mixture is packed into size 3 hard gelatin capsules in a capsule filling machine.

Example 5: Tablets containing 2.5mg, 5mg, 10mg, 25mg, 50mg of active substance

| **Active substance** | 2.5 mg Mg/per tablet | 5 mg Mg/per tablet | 10 mg Mg/per tablet | 25 mg Mg/per tablet | 50 mg Mg/per tablet |
|---|---|---|---|---|---|
| **Wet granulation** | | | | | |
| active substance | 2.5000 | 5.000 | 10.00 | 25.00 | 50.00 |
| Lactose Monohydrate | 40.6250 | 81.250 | 162.50 | 113.00 | 226.00 |
| Microcrystalline Cellulose | 12.5000 | 25.000 | 50.00 | 40.00 | 80.00 |
| Hydroxypropyl | 1.8750 | 3.750 | 7.50 | 6.00 | 12.00 |
| Cellulose | | | | | |
| Croscarmellose Sodium | 1.2500 | 2.500 | 5.00 | 4.00 | 8.00 |
| Purified Water | q.s. | q.s. | q.s. | q.s. | q.s. |
| **Dry Adds** | | | | | |
| Microcrystalline Cellulose | 3.1250 | 6.250 | 12.50 | 10.00 | 20.00 |
| Colloidal silicon dioxide | 0.3125 | 0.625 | 1.25 | 1.00 | 2.00 |
| Magnesium stearate | 0.3125 | 0.625 | 1.25 | 1.00 | 2.00 |
| Total core | 62.5000 | 125.000 | 250.00 | 200.00 | 400.00 |
| **Film Coating** | | | | | |
| Film coating system | 2.5000 | 4.000 | 7.00 | 6.00 | 9.00 |
| Purified Water | q.s. | q.s. | q.s. | q.s. | q.s. |
| **Total** | **65.000** | **129.000** | **257.00** | **206.00** | **409.00** |

Example 6: Manufacturing process for tablets

| Step | EQUIPMENT | MATERIALS | OPERATION | IN-PROCESS CONTROLS |
|---|---|---|---|---|
| 1 | Screen, blender and high shear granulator | Active substance → | MIX | |
| | | Hydroxypropyl Cellulose (screened) → | | |
| | | Croscarmellose Sodium → | | |
| | | Part of Microcrystalline Cellulose (PH102) → | | |
| | | Lactose Monohydrate → | | |
| 2 | High shear granulator | Purified Water → | GRANULATE ↓ | |
| 3 | Fluid bed drier | | DISCHARGE ONTO DRYER AND DRY ↓ | LOD ≤ 2.0% at 100°C |

| | | | | | |
|---|---|---|---|---|---|
| 4 | Mill | | | DRY MILL ↓ | |
| 5 | Mill, blender | Colloidal Silicon Dioxide + Microcrystalline Cellulose (PH102) | → | MIX ↓ | |
| 6 | Mill, blender | Magnesium Stearate | → | MIX ↓ | |
| | | | | Final tablet blend ↓ | |
| 7 | Tablet press | | | COMPRESS INTO TABLETS ↓ | Tablet weight, height, crushing strength, friability, disintegration |
| | | | | Core tablets ↓ | |
| 8 | Propeller Stirrer Drum coater | Suspend film-coating system in water and mix | → | FILM COATING ↓ | |
| | | | | Final film coated tablets | Tablet weight, height, crushing strength, disintegration |

Example 7: Pharmaceutical composition containing other fillers

Copovidone is dissolved in purified water at ambient temperature to produce a granulation liquid. A glucopyranosyl-substituted benzene derivative according to the present invention, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

Magnesium stearate is passed through a sieve for delumping and added to the granulate. Subsequently the final blend is produced by final blending in a suitable blender for three minutes and compressed into tablet cores.

Hydroxypropyl methylcellulose, polyethylene glycol, talc, titanium dioxide and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| **Ingredient** | **mg/ tablet** | **mg/ tablet** | **mg/ tablet** | **mg/ tablet** | **mg/ tablet** |
|---|---|---|---|---|---|
| Active substance | 2.5 | 5.0 | 10.0 | 25.0 | 50.0 |
| Mannitol | 133.4 | 130.9 | 125.9 | 110.9 | 221.8 |
| Pregelatinised starch | 18.0 | 18.0 | 18.0 | 18.0 | 36.0 |
| Maize starch | 18.0 | 18.0 | 18.0 | 18.0 | 36.0 |
| Copovidone | 5.4 | 5.4 | 5.4 | 5.4 | 10.8 |

(continued)

| Ingredient | mg/ tablet | mg/ tablet | mg/ tablet | mg/ tablet | mg/ tablet |
|---|---|---|---|---|---|
| Magnesium stearate | 2.7 | 2.7 | 2.7 | 2.7 | 5.4 |
| Film coat | 5.0 | 5.0 | 5.0 | 5.0 | 10.0 |
| **Total** | **185.0** | **185.0** | **185.0** | **185.0** | **370.0** |

Example 8: Pharmaceutical composition containg other disintegrant

Copovidone is dissolved in purified water at ambient temperature to produce a granulation liquid. An glucopyranosyl-substituted benzene derivative according to the present invention, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

Crospovidone is added to the dried granulate and mixed for 5 minutes to produce the main blend. Magnesium stearate is passed through a sieve for delumping and added to main blend. Subsequently the final blend is produced by final blending in a suitable blender for three minutes and compressed into 8 mm round tablet cores with a compression force of 16 kN.

Hydroxypropyl methylcellulose, polyethylene glycol, talc, titanium dioxide and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| Ingredient | mg/ tablet | mg/ tablet | mg/ tablet | mg/ tablet | mg/ tablet |
|---|---|---|---|---|---|
| Active substance | 2.5 | 5.0 | 10.0 | 25.0 | 50.0 |
| Mannitol | 127.5 | 125.0 | 120.0 | 105.0 | 210.0 |
| Microcrystalline Cellulose | 39.0 | 39.0 | 39.0 | 39.0 | 78.0 |
| Crospovidone | 2.0 | 2.0 | 2.0 | 2.0 | 4.0 |
| Copovidone | 5.4 | 5.4 | 5.4 | 5.4 | 10.8 |
| Magnesium stearate | 3.6 | 3.6 | 3.6 | 3.6 | 7.2 |
| Film coat | 5.0 | 5.0 | 5.0 | 5.0 | 10.0 |
| **Total** | **185.0** | **185.0** | **185.0** | **185.0** | **370.0** |

The tablet hardness, the friability, the content uniformity, the disintegration time and the dissolution properties are determined as described hereinbefore.

Example 9: Direct compression formulation

1. Screen the active ingredient, microcrystalline cellulose, croscarmellose.sodium and either hydroxypropyl cellulose or polyethylene glycol powder through a 20 mesh hand screen.
2. Add the above items into the high shear mixer and mix for two minutes.
3. Make a premix (-1/1) of the lactose and colloidal silicon dioxide.
4. Screen the premix through a 20 mesh hand screen and add to the mixer.
5. Screen the remaining lactose through a 20 mesh hand screen and add to the mixer.
6. Mix in components in the mixer for 2 minutes.
7. Screen the magnesium stearate through a 30 mesh hand screen and add to the mixer.
8. Mix for 1 minute 30 seconds to obtain the final blend.

9 Tabletting of the final blend on a suitable tabletting press.

10. Optionally film coating of the tablet cores.

| Ingredient | mg/ tablet | mg/ tablet | mg/ tablet | mg/ tablet | mg/ tablet |
|---|---|---|---|---|---|
| Active substance | 2.5000 | 5.000 | 10.00 | 25.0 | 50.0 |
| Lactose Monohydrate | 43.7500 | 87.500 | 175.00 | 74.0 | 148.0 |
| Microcrystalline Cellulose | 12.5000 | 25.000 | 50.00 | 80.0 | 160.0 |
| Polyethylene glycol | - | - | - | 10.0 | 20.0 |
| Croscarmellose sodium | 1.2500 | 2.500 | 5.00 | 8.0 | 16.0 |
| Hydroxypropyl cellulose | 1.8750 | 3.750 | 7.50 | - | - |
| Colloidal Silicon dioxide | 0.3125 | 0.625 | 1.25 | 1.0 | 2.0 |
| Magnesium stearate | 0.3125 | 0.625 | 1.25 | 2.0 | 4.0 |
| Film coat | 2.5000 | 4.000 | 7.00 | 6.00 | 9.00 |
| Purified water | q.s. | q.s. | q.s. | q.s. | q.s. |
| **Total** | **65.000** | **129.000** | **257.00** | **206.00** | **409.00** |

Example 10: Tablets containing 0.5mg, 5mg, 25mg, 100mg of active substance

| **Active substance** | 0.5 mg mg/per tablet | 5 mg mg/per tablet | 25 mg mg/per tablet | 100 mg mg/per tablet |
|---|---|---|---|---|
| **Wet granulation** | | | | |
| active substance | 2.5000 | 5.000 | 25.00 | 100.00 |
| Lactose Monohydrate | 60.00 | 55.00 | 42.00 | 168.00 |
| Microcrystalline Cellulose | 20.00 | 20.00 | 38.00 | 152.00 |
| Hyd roxypropyl Cellulose | 5.00 | 5.00 | 7.50 | 30.00 |
| Croscarmellose Sodium | 4.00 | 4.00 | 6.00 | 24.00 |
| Purified Water | q.s. | q.s. | q.s. | q.s. |
| **Dry Adds** | | | | |
| Microcrystalline Cellulose | 10.00 | 10.00 | 30.00 | 120.00 |
| Colloidal silicon dioxide | - | 0.50 | 0.75 | 3.00 |
| Magnesium stearate | 0.50 | 0.50 | 0.75 | 3.00 |
| **Total** | **100.00** | **100.00** | **150.00** | **600.00** |

The active substance, e.g. the compound (1.9), preferably in the crystalline form (I.9X), hydroxypropyl cellulose, and croscarmellose sodium are mixed in a blender. This premix is mixed with lactose monohydrate and a portion of microcrystalline cellulose. The resulting blend is granulated with purified water. Multiple granulation subparts may be produced for an individual tablet batch, as needed, depending on the batch size and equipment used.

The granulation is discharged onto dryer trays and dried. The granulation is then milled. The remainder of the microcrystalline cellulose is added (as a premix with the colloidal silicon dioxide for all strengths other than the 0.5 mg) to the milled granulation, and mixed. The magnesium stearate is premixed with a portion of the blend, screened into the remainder of the granulation, and mixed.

The final tablet blend is compressed into tablets using a tablet press. The finished tablets are packaged using a suitable container closure system.

Example 11: Tablets containing 1mg, 5mg, 25mg of active substance

| **Active substance** | 1 mg mg/per tablet | 5 mg mg/per tablet | 25 mg mg/per tablet |
|---|---|---|---|
| **Wet granulation** | | | |
| active substance | 1.00 | 5.00 | 25.00 |
| Lactose Monohydrate | 63.00 | 59.00 | 39.00 |
| Microcrystalline Cellulose | 20.00 | 20.00 | 20.00 |
| Hyd roxypropyl Cellulose | 3.00 | 3.00 | 3.00 |
| Croscarmellose Sodium | 2.00 | 2.00 | 2.00 |
| Purified Water | q.s. | q.s. | q.s. |
| **Dry Adds** | | | |
| Microcrystalline Cellulose | 10.00 | 10.00 | 10.00 |
| Colloidal silicon dioxide | 0.50 | 0.50 | 0.50 |
| Magnesium stearate | 0.50 | 0.50 | 0.50 |
| **Total** | **100.00** | **100.00** | **100.00** |

The active substance, i.e. the compound (1.9), preferably in the crystalline form (I.9X), is passed through a screen and added to a blender or a high shear granulator. The hydroxypropyl cellulose and croscarmellose sodium are passed through a screen, added to the drug substance, and mixed. The intra-granular portion of microcrystalline cellulose is passed through a screen into a high shear granulator and mixed with the drug substance premix. Lactose is then added by passing the material through a screen into the granulator and mixing. The resulting blend is granulated with purified water. For larger batches, multiple granulation subparts may be produced for an individual tablet batch, as needed, depending on the batch size and equipment used.

The granulation is discharged onto dryer trays and dried. The granulation is then passed through a mill into a blender. The colloidal silicon dioxide is pre-mixed with a portion of the extra-granular microcrystalline cellulose. This premix is passed through a mill into the blender, followed by the remaining extra-granular microcrystalline cellulose, and mixed with the milled granulation. The magnesium stearate is premixed with a portion of the blend, passed through a mill into the remainder of the granulation, and mixed.

The final tablet blend is compressed into tablets using a tablet press. The finished tablets are packaged using a suitable container closure system.

**Examples of Tests with regard to Properties of Pharmaceutical Compositions and Pharmaceutical Dosage Forms**

1. Disintegration Test

Disintegration test was performed as described in USP31-NF26 S2, chapter 701 (disintegration).

2. Dissolution Test

The standard dissolution test is described in USP31-NF26 S2, chapter 711 (dissolution). The paddle method (Apparatus 2) with an agitation speed of 50 rpm was used. The dissolution media is 900 mL 0.05 M Potassium phosphate buffer pH 6.8 at a temperature of 37°C. Samples are taken after 10, 15, 20, 30 and 45 minutes. The samples are analyzed via HPLC.

3. Particle Size Distribution Measurement by Laser Diffraction

Particle size distribution measurement is performed for example via light scattering or laser diffraction technique. To determine the particle size the powder is fed into a laser diffraction spectrometer for example by means of a dispersing unit. The test method is described below in detail:

| | |
|---|---|
| Equipment: | Laser Diffraction Spectrometer Sympatec HELOS Particle Sizer. |
| Lens: | R31 (0.5/0.9μm - 175μm) |
| Sample Dispersing Unit: | Dry disperser RODOS/M |
| Vacuum: | Nilfisk |
| Feeder: | ASPIROS |
| Feed Velocity: | 60.00 mm/s |
| Primary pressure: | 2.00 bar |
| Injector depression: | maximize (mbar)2 |
| Reference Measurement: | 10 seconds |
| Cycle Time: | 100 msec |
| Trigger Conditions: | Start 0.0 seconds after optical concentration ≥ 1% valid always |

Stop after 5.0 seconds optical concentration ≤ 1% or after 30 seconds real time

| | |
|---|---|
| Optical Concentration: | Approximately range 3 - 12 % |
| Evaluation: | HRLD |
| Sample Size: | Approximately 100 mg |
| Number of measuremtns: | 2 (duplicate) |

The instrument is set up according to the manufacturer's recommendation and using the manufacturer provided software. The sample container is thoroughly mixed and tumbled prior to removing a portion of the sample to ensure that a representative sample is tested. Duplicate samples are prepared by using a spatula to transfer approximately 100 mg of a sample into the ASPIROS glass vials and cap the vials. The capped cials are placed into the feeder.

4. Tablet hardness and friability

Tablet hardness and friability test was performed as described in USP31-NF26 S2, chapter 1217 (tablet breaking force).

**Example: Pharmaceutical composition of compound (1.9) and linagliptin**

In the following an example of pharmaceutical compositions comprising the compound (1.9) (API 1) and linagliptin (API 2) and their manufacture are provided. The active pharmaceutical ingredients API 1 and API 2 are processed in one granulation step and a one-layer tablet is pressed. At the beginning copovidone is dissolved in purified water at ambient temperature (about 20°C) to produce a granulation liquid. API 1, API 2, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.
Crospovidone and talc are added to the dried granulate and mixed for 5 minutes to produce the main blend. Magnesium stearate is passed through a sieve for delumping and added to main blend. Subsequently the final blend is produced by final blending in a suitable blender for three minutes and compressed into 8 mm round tablet cores with a compression force of 16 kN.

Hydroxypropyl methylcellulose, polyethylene glycol, talc, titanium dioxide, mannitol and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| Ingredient | mg/ tablet | mg/ tablet | mg/ tablet | mg/ tablet | mg / tablet |
|---|---|---|---|---|---|
| API 1 | 2.5 | 5.0 | 10.0 | 25.0 | 50.0 |
| API 2 | 5.0 | 5.0 | 5.0 | 5.0 | 5.0 |
| Mannitol | 113.55 | 111.05 | 106.05 | 91.05 | 66.05 |
| Pregelatinised starch | 18.0 | 18.0 | 18.0 | 18.0 | 18.0 |
| Maize starch | 19.8 | 19.8 | 19.8 | 19.8 | 19.8 |

(continued)

| Ingredient | mg/ tablet | mg/ tablet | mg/ tablet | mg/ tablet | mg / tablet |
|---|---|---|---|---|---|
| Crospovidone | 3.6 | 3.6 | 3.6 | 3.6 | 3.6 |
| Copovidone | 5.4 | 5.4 | 5.4 | 5.4 | 5.4 |
| Talc | 9.0 | 9.0 | 9.0 | 9.0 | 9.0 |
| Magnesium stearate | 3.15 | 3.15 | 3.15 | 3.15 | 3.15 |
| Hydoxypropyl methylcellulose | 1.7500 | 1.7500 | 1.7500 | 1.7500 | 1.7500 |
| Polyethylene glycol | 0.6000 | 0.6000 | 0.6000 | 0.6000 | 0.6000 |
| Iron oxides | 0.0125 | 0.0125 | 0.0125 | 0.0125 | 0.0125 |
| Titanium dioxide | 0.7375 | 0.7375 | 0.7375 | 0.7375 | 0.7375 |
| Talc | 0.9000 | 0.9000 | 0.9000 | 0.9000 | 0.9000 |
| Mannitol | 1.0000 | 1.0000 | 1.0000 | 1.0000 | 1.0000 |
| Total | 185.0 | 185.0 | 185.0 | 185.0 | 185.0 |

## Claims

**1.** A pharmaceutical composition comprising the SGLT2 inhibitor 1-chloro-4-(β-D-glucopyranos-1-yl)-2-[4-((*S*)-tetrahydrofuran-3-yloxy)-benzyl]-benzene for use in a method for preventing, slowing the progression of, delaying or treating renal hyperfiltrative injury in a patient diagnosed with type 1 diabetes mellitus in need thereof.

**2.** A pharmaceutical composition comprising the SGLT2 inhibitor 1-chloro-4-(β-D-glucopyranos-1-yl)-2-[4-((*S*)-tetrahydrofuran-3-yloxy)-benzyl]-benzene for use in a method for preventing, slowing the progression of, delaying or treating renal hyperfiltration in a patient diagnosed with type 1 diabetes mellitus.

**3.** The pharmaceutical composition for use in a method according to claim 1 or 2, wherein the patient is an individual diagnosed or showing one or more of the following conditions:

(a) diabetes mellitus;
(b) congenital or acquired obstructive uro/nephropathy;
(c) progressive chronic kidney disease (CKD);
(d) acute renal failure (ARF);
(e) renal transplant recipients;
(f) renal transplant donors; or
(g) unilateral total or partial nephrectomized patients.

**4.** The pharmaceutical composition for use in a method according to any one of the preceding claims, wherein the patient has an GFR equal to or greater than 125 mL/min/1.73 $m^2$.

**5.** The pharmaceutical composition for use in a method according to any one of the preceding claims, wherein the patient has an GFR equal to or greater than 140 mL/min/1.73 $m^2$.

**6.** The pharmaceutical composition for use in a method according to any one of the preceding claims, wherein the patient:

(1) is an individual diagnosed of one or more of the conditions selected from the group consisting of overweight, obesity, visceral obesity and abdominal obesity; or
(2) is an individual who shows one, two or more of the following conditions:

(a) a fasting blood glucose or serum glucose concentration greater than 100 mg/dL, in particular greater than 125 mg/dL;
(b) a postprandial plasma glucose equal to or greater than 140 mg/dL;
(c) an HbA1c value equal to or greater than 6.0 %, equal to or greater than 6.5%, or equal to or greater

than 8.0 %; or

(3) is an individual wherein one, two, three or more of the following conditions are present:

(a) obesity, visceral obesity and/or abdominal obesity,
(b) triglyceride blood level ≥ 150 mg/dL,
(c) HDL-cholesterol blood level < 40 mg/dL in female patients and < 50 mg/dL in male patients,
(d) a systolic blood pressure ≥ 130 mm Hg and a diastolic blood pressure ≥ 85 mm Hg,
(e) a fasting blood glucose level ≥ 100 mg/dL; or

(4) is an individual with morbid obesity.

**7.** The pharmaceutical composition for use in a method according to any one of the preceding claims, wherein the pharmaceutical composition additionally comprises one or more pharmaceutically acceptable carriers.

**8.** The pharmaceutical composition for use in a method according to any one of the preceding claims, wherein the pharmaceutical composition comprises the SGLT-2 inhibitor in a range from 1 mg to 25 mg.

**9.** The pharmaceutical composition for use in a method according to any one of the preceding claims, wherein the pharmaceutical composition comprises 1 mg, 2.5 mg, 5 mg, 7.5 mg, 10 mg, 12.5 mg, 15 mg, 20 mg or 25 mg of the SGLT-2 inhibitor.

**Patentansprüche**

**1.** Pharmazeutische Zusammensetzung, umfassend den SGLT2-Inhibitor 1-Chlor-4-(β-D-glucopyranos-1-yl)-2-[4-((*S*)-tetrahydrofuran-3-yloxy)-benzyl]-benzol zur Verwendung in einem Verfahren zum Vorbeugen, zum Verlangsamen des Fortschreitens, zum Verzögern oder Behandeln eines Nierenhyperfiltrationsschadens in einem Patienten mit Bedarf hierfür, bei dem Diabetes mellitus Typ 1 diagnostiziert wurde.

**2.** Pharmazeutische Zusammensetzung, umfassend den SGLT2-Inhibitor 1-Chlor-4-(β-D-glucopyranos-1-yl)-2-[4-((*S*)-tetrahydrofuran-3-yloxy)-benzyl]-benzol zur Verwendung in einem Verfahren zum Vorbeugen, zum Verlangsamen des Fortschreitens, Verzögern oder Behandeln von Nierenhyperfiltration in einem Patienten, bei dem Diabetes mellitus Typ 1 diagnostiziert wurde.

**3.** Pharmazeutische Zusammensetzung zur Verwendung in einem Verfahren nach Anspruch 1 oder 2, wobei der Patient ein Individuum darstellt, bei dem einer oder mehrere der folgenden Zustände diagnostiziert wurden oder der einen oder mehrere der folgenden Zustände zeigt:

(a) Diabetes mellitus;
(b) angeborene oder erworbene obstruktive Uro-/Nephropathie;
(c) progressive chronische Nierenerkrankung (CKD);
(d) akutes Nierenversagen (ARF);
(e) Nierentransplantatempfänger;
(f) Nierentransplantatspender; oder
(g) unilateral total oder partiell nephrektomierte Patienten.

**4.** Pharmazeutische Zusammensetzung zur Verwendung in einem Verfahren nach irgendeinem der vorhergehenden Ansprüche, wobei der Patient einen GFR größer oder gleich 125 mL/min/1,73 $m^2$ aufweist.

**5.** Pharmazeutische Zusammensetzung zur Verwendung in einem Verfahren nach irgendeinem der vorhergehenden Ansprüche, wobei der Patient einen GFR größer oder gleich 140 mL/min/1,73 $m^2$ aufweist.

**6.** Pharmazeutische Zusammensetzung zur Verwendung in einem Verfahren nach irgendeinem der vorhergehenden Ansprüche, wobei der Patient:

(1) ein Individuum ist, bei dem einer oder mehrere der Zustände diagnostiziert wurden, ausgewählt aus der Gruppe, bestehend aus Übergewicht, Adipositas, viszeraler Adipositas und abdominaler Adipositas; oder

(2) ein Individuum ist, das ein, zwei oder mehr der folgenden Zustände bzw. Bedingungen zeigt:

(a) eine Nüchternglucose- oder Serum-Glucosekonzentration größer 100 mg/dL, insbesondere größer 125 mg/dL;
(b) eine postprandiale Plasma-Glucose größer oder gleich 140 mg/dL;
(c) einen HbA1c-Wert größer oder gleich 6,0 %, größer oder gleich 6,5% oder größer oder gleich 8,0 %; oder

(3) ein Individuum ist, bei dem ein, zwei, drei oder mehr der folgenden Zustände bzw. Bedingungen vorliegen:

(a) Adipositas, viszerale Adipositas und/oder abdominale Adipositas,
(b) Triglyceridblutspiegel ≥ 150 mg/dL,
(c) HDL-Cholesterol-Blutspiegel < 40 mg/dL bei weiblichen Patienten und < 50 mg/dL bei männlichen Patienten,
(d) einen systolischen Blutdruck ≥ 130 mm Hg und einen diastolischen Blutdruck ≥ 85 mm Hg,
(e) einen Nüchternblutglucosespiegel ≥ 100 mg/dL; oder

(4) ein Individuum mit morbider Adipositas.

**7.** Pharmazeutische Zusammensetzung zur Verwendung in einem Verfahren nach irgendeinem der vorhergehenden Ansprüche, wobei die pharmazeutische Zusammensetzung zusätzlich einen oder mehrere pharmazeutisch akzeptable bzw. annehmbare Träger umfasst.

**8.** Pharmazeutische Zusammensetzung zur Verwendung in einem Verfahren nach irgendeinem der vorhergehenden Ansprüche, wobei die pharmazeutische Zusammensetzung den SGLT-2-Inhibitor in einem Bereich von 1 mg bis 25 mg umfasst.

**9.** Pharmazeutische Zusammensetzung zur Verwendung in einem Verfahren nach irgendeinem der vorhergehenden Ansprüche, wobei die pharmazeutische Zusammensetzung 1 mg, 2,5 mg, 5 mg, 7,5 mg, 10 mg, 12,5 mg, 15 mg, 20 mg oder 25 mg des SGLT-2-Inhibitors umfasst.

## Revendications

**1.** Composition pharmaceutique comprenant l'inhibiteur de SGLT2 1-chloro-4-(β-D-glucopyranos-1-yl)-2-[4-(*(S)*-tétrahydrofuran-3-yloxy)-benzyl]-benzène pour son utilisation dans une méthode de prévention, de ralentissement de la progression, de retardement ou de traitement d'une lésion d'hyperfiltration rénale chez un patient le nécessitant à qui un diabète sucré de type 1 a été diagnostiqué.

**2.** Composition pharmaceutique comprenant l'inhibiteur de SGLT2 1-chloro-4-(β-D-glucopyranos-1-yl)-2-[4-(*(S)*-tétrahydrofuran-3-yloxy)-benzyl]-benzène pour son utilisation dans une méthode de prévention, de ralentissement de la progression, de retardement ou de traitement de l'hyperfiltration rénale chez un patient à qui un diabète sucré de type 1 a été diagnostiqué.

**3.** Composition pharmaceutique pour son utilisation dans une méthode selon la revendication 1 ou 2, dans laquelle le patient est un individu chez qui ont été diagnostiquées ou présentant une ou plusieurs des affections suivantes :

(a) diabète sucré ;
(b) uro/néphropathie obstructive congénitale ou acquise ;
(c) insuffisance rénale chronique progressive (CKD) ;
(d) insuffisance rénale aiguë (IRA) ;
(e) receveurs de transplantation rénale ;
(f) donneurs de rein ; ou
(g) patients ayant subi une néphrectomie unilatérale totale ou partielle.

**4.** Composition pharmaceutique pour son utilisation dans une méthode selon l'une quelconque des revendications précédentes, dans laquelle le patient présente un DFG supérieur ou égal à 125 ml/min/1,73 $m^2$.

**5.** Composition pharmaceutique pour son utilisation dans une méthode selon l'une quelconque des revendications

précédentes, dans laquelle le patient présente un DFG supérieur ou égal à 140 ml/min/1,73 $m^2$.

**6.** Composition pharmaceutique pour son utilisation dans une méthode selon l'une quelconque des revendications précédentes, dans laquelle le patient :

(1) est un individu chez qui ont été diagnostiquées une ou plusieurs des affections sélectionnées dans le groupe consistant en le surpoids, l'obésité, l'obésité viscérale et l'obésité abdominale ; ou
(2) est un individu qui présente une, deux ou plus des affections suivantes :

(a) une glycémie ou concentration de glucose sérique à jeun supérieure à 100 mg/dl, en particulier supérieure à 125 mg/dl ;
(b) un glucose plasmatique postprandial supérieur ou égal à 140 mg/dl ;
(c) une valeur d'HbAlc supérieure ou égale à 6,0 %, supérieure ou égale à 6,5 %, ou supérieure ou égale à 8,0 % ; ou

(3) est un individu chez qui une, deux, trois ou plus des affections suivantes sont présentes :

(a) l'obésité, l'obésité viscérale et/ou l'obésité abdominale ;
(b) un niveau de triglycérides dans le sang ≥ 150 mg/dl,
(c) une cholestérolémie des HDL < 40 mg/dl chez les patientes de sexe féminin et < 50 mg/dl chez les patients de sexe masculin,
(d) une pression artérielle systolique ≥ 130 mm Hg et une pression artérielle diastolique ≥ 85 mm Hg,
(e) un niveau de glycémie à jeun ≥ 100 mg/dL ; ou

(4) est un individu présentant une obésité morbide.

**7.** Composition pharmaceutique pour son utilisation dans une méthode selon l'une quelconque des revendications précédentes, dans laquelle la composition pharmaceutique comprend en outre un ou plusieurs supports pharmaceutiquement acceptables.

**8.** Composition pharmaceutique pour son utilisation dans une méthode selon l'une quelconque des revendications précédentes, dans laquelle la composition pharmaceutique comprend l'inhibiteur de SGLT-2 dans une plage de 1 mg à 25 mg.

**9.** Composition pharmaceutique pour son utilisation dans une méthode selon l'une quelconque des revendications précédentes, dans laquelle la composition pharmaceutique comprend 1 mg, 2,5 mg, 5 mg, 7,5 mg, 10 mg, 12,5 mg, 15 mg, 20 mg ou 25 mg de l'inhibiteur de SGLT-2.

Figure 1: X-ray powder diffraction pattern of the crystalline form

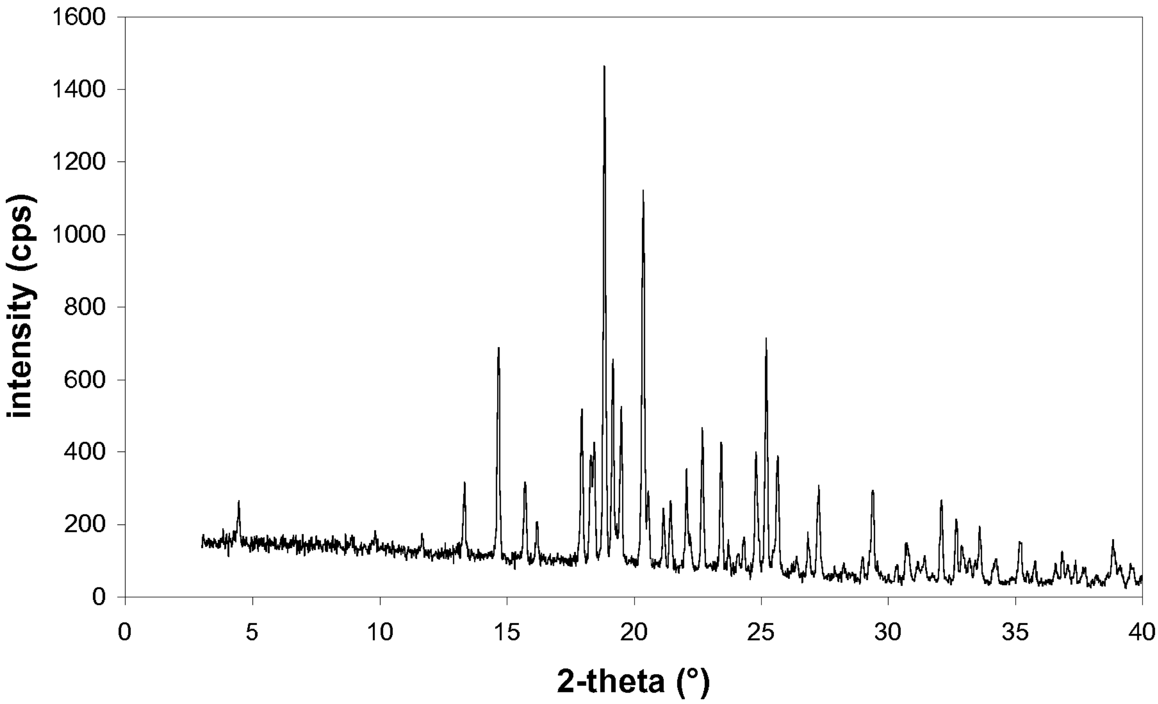


Figure 2: DSC and TG diagram of the crystalline form

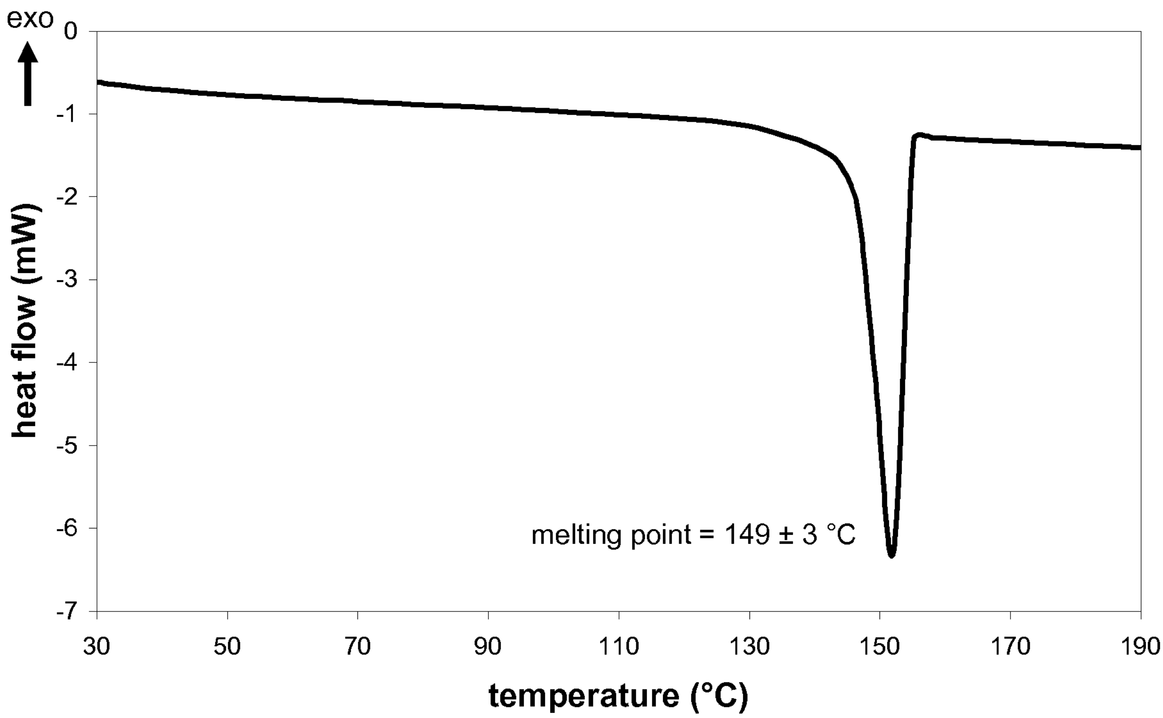

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008055940 A2
- WO 2005092877 A
- WO 2007093610 A
- WO 0127128 A
- WO 03099836 A
- WO 2006034489 A
- WO 2006064033 A
- WO 2006117359 A
- WO 2006117360 A
- WO 2007025943 A
- WO 2007028814 A
- WO 2007031548 A
- WO 2007128749 A
- WO 2008049923 A
- WO 2008055870 A
- WO 2008055940 A
- WO 2006120208 A
- WO 2011039107 A
- WO 2011039108 A
- WO 2011039337 A
- WO 2009022007 A
- WO 2010092124 A
- WO 2010092125 A
- WO 2006124529 A
- WO 2009020802 A


### Non-patent literature cited in the description


- **MAGEE et al.** *Diabetologia,* 2009, vol. 52, 691-697
- **VALLON et al.** *Journal of the American Society of Nephrology,* 1999, vol. 10 (12), 2569-2576
- **YAO et al.** *J. Med. Chem.,* 2011, vol. 54, 166-178
- **MELSOM et al.** *Diabetes Care,* 2011
- Joint scientific statement (IDF, NHLBI, AHA, WHO, IAS, IASO). *Circulation,* 2009, vol. 120, 1640-1645
- World Health Organization. Obesity: Preventing and Managing the Global Epidemic: Report on a WHO Consultation. *World Health Organ Tech Rep Ser.,* 2000, vol. 894, 1-253
- **FORD ES et al.** *JAMA,* 2002, vol. 287, 356-9
- **KATSUKI A et al.** *Diabetes Care,* 2001, vol. 24, 362-5
- **MATTHEWS et al.** *Diabetologia,* 1985, vol. 28, 412-19
- **FORST et al.** *Diabetes,* 2003, vol. 52 (1), A459
- **GALVIN P et al.** *Diabet Med,* 1992, vol. 9, 921-8
- **STUMVOLL et al.** *Eur J Clin Invest,* 2001, vol. 31, 380-81
- **J. B. MEIGS et al.** *Diabetes,* 2003, vol. 52, 1475-1484
- The Prevention or Delay of Type 2 Diabetes. Diabetes Care. American Diabetes Association and the National Institute of Diabetes and Digestive and Kidney Diseases, 2002, vol. 25, 742-749
- **LAAKSONEN DE et al.** *Am J Epidemiol,* 2002, vol. 156, 1070-7
- Executive Summary of the Third Report of the National Cholesterol Education Program (NCEP) Expert Panel on Detection, Evaluation, and Treatment of High Blood Cholesterol in Adults (Adult Treatment Panel III). *JAMA: Journal of the American Medical Association,* 2001, vol. 285, 2486-2497
- **LAAKSONEN DE et al.** *Am J Epidemiol.,* 2002, vol. 156, 1070-7
- Labor und Diagnose. TH-Books Verlagsgesellschaft mbH, 2000
- *European Directorate for the Quality of Medicines and Health Care,* 2000